(19) Europäisches Patentamt
European Patent Office
Office européen des brevets

(11) **EP 2 916 804 B1**

(12) **EUROPEAN PATENT SPECIFICATION**

(45) Date of publication and mention of the grant of the patent:
**25.12.2019 Bulletin 2019/52**

(21) Application number: **13789776.5**

(22) Date of filing: **08.11.2013**

(51) Int Cl.:
***A61K 8/365*** *(2006.01)* ***A61Q 5/04*** *(2006.01)*
***A61K 8/31*** *(2006.01)* ***A61K 8/34*** *(2006.01)*
***A61K 8/37*** *(2006.01)* ***A61K 8/92*** *(2006.01)*
***A61K 8/24*** *(2006.01)* ***A61K 8/362*** *(2006.01)*
***A61K 8/368*** *(2006.01)* ***A61K 8/36*** *(2006.01)*

(86) International application number:
**PCT/EP2013/073448**

(87) International publication number:
**WO 2014/072490 (15.05.2014 Gazette 2014/20)**

(54) **COMPOSITION COMPRISING A DICARBONYL COMPOUND AND AN ACID, THE PROCESS FOR STRAIGHTENING KERATIN FIBRES USING THIS COMPOSITION**

ZUSAMMENSETZUNG MIT EINER DICARBONYLVERBINDUNG UND EINER SÄURE, VERFAHREN ZUR GLÄTTUNG VON KERATINFASERN UNTER VERWENDUNG DIESER ZUSAMMENSETZUNG

COMPOSITION COMPRENANT UN COMPOSÉ DICARBONYLE ET UN ACIDE ET PROCÉDÉ POUR LE LISSAGE DE FIBRES DE KÉRATINE UTILISANT CETTE COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2012 FR 1260662**

(43) Date of publication of application:
**16.09.2015 Bulletin 2015/38**

(73) Proprietor: **L'Oréal**
**75008 Paris (FR)**

(72) Inventor: **DAUBRESSE, Nicolas**
**F-78170 La Celle Saint Cloud (FR)**

(74) Representative: **L'Oreal**
**Service D.I.P.I.**
**9, rue Pierre Dreyfus**
**92110 Clichy (FR)**

(56) References cited:
**WO-A1-2007/135299** **WO-A1-2012/105985**
**WO-A2-2011/104282** **WO-A2-2012/010351**
**US-A1- 2011 144 141**

- **DATABASE GNPD [Online] MINTEL; February 2012 (2012-02), "Moroccan Relaxing Treatment with Argan Oil", XP002703715, Database accession no. 1692744**
- **DATABASE GNPD [Online] MINTEL; October 2012 (2012-10), "Luxury Keratin Treatment", XP002703716, Database accession no. 1892615**
- **DATABASE GNPD [Online] MINTEL; January 2012 (2012-01), "Reduce Kit'30", XP002703717, Database accession no. 1704182**

**EP 2 916 804 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a cosmetic composition, in particular hair composition, based on one or more particular dicarbonyl compounds and/or derivatives thereof and/or hydrates thereof and/or salts thereof i), and on one or more acids different from i), in particular carboxylic acids, different from said particular dicarbonyl compound(s) and also to a process for straightening the hair using this composition.

In the hair field, consumers wish to have available compositions which make it possible to introduce a temporary change to their head of hair, while targeting a good wear property for the effect produced. In general, it is desirable for the change to withstand shampooing operations for a minimum of 15 days, indeed even more, depending on the nature of said change.

Treatments already exist for modifying the colour or the shape of the hair and also, to some extent, the texture of the hair. One of the known treatments for modifying the texture of the hair consists of the combination of heat and of a formaldehyde-comprising composition. This treatment is in particular effective for conferring a better appearance on damaged hair and/or for treating long hair and curly hair.

The action of formaldehyde is associated with its ability to crosslink proteins by reaction with its nucleophilic sites. The heat used can be that of an iron (flat tongs or crimping iron), the temperature of which can generally reach 200°C or more. However, there is an increasing desire to avoid the use of such substances, which can prove to be aggressive to the hair and other keratin materials.

Application WO2011/104282 has thus provided a novel process for semi-permanently straightening the hair which consists in applying an alpha-keto acid solution to the hair for 15 to 120 minutes, in then drying and, finally, in straightening the head of hair with an iron at a temperature of approximately 200°C. The alpha-keto acid or $\alpha$-keto acid used is preferably glyoxylic acid.

WO2012/105985 discloses a process for straightening the hair which consists in applying an alkaline composition comprising a base, and then, after drying the hair, applying an acid composition of pH less than 1.5 comprising a compound which may be acid glyoxylic amide or a glyoxylic amide, these applications being followed by an iron passage.

However, it has been noted that the use of glyoxylic acid can result in some significant limitations; in particular, at high concentration it may not be well tolerated, in particular when the scalp is sensitive and/or irritated. Its volatility, enhanced by the use of heat (iron), can also present a problem. Furthermore, cosmetic formulations having an acidic pH can detrimentally affect the hair and/or detrimentally affect the colour thereof.

It is already known practice to use glyoxylic acid esters in hair compositions, in particular in hair dyeing compositions, as described in document DE19859722, and in reducing compositions, as described in document DE19860239.

However, the efficiency of these compounds is not yet sufficient.

The purpose of the invention is to develop a straightening/relaxing composition which is stable over time and which makes it possible to straighten/relax and/or reduce the volume of the hair in an efficient and persistent manner while limiting damage to the hair, while at the same time retaining comfort at the time of application for the user of the composition, but also for the hairdresser who applies it.

Thus, a subject of the present invention is a cosmetic composition comprising:

i) one or more dicarbonyl compounds corresponding to formula (I) below, and/or derivatives thereof and/or hydrates thereof and/or salts thereof:

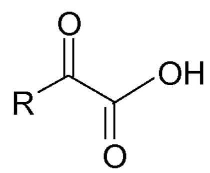

**(I)**

in which formula **(I):**

**R** represents an atom or group chosen from i) hydrogen, ii) carboxyl -C(O)-OH, iii) linear or branched $C_1$-$C_6$ alkyl which is optionally substituted, preferably with at least one hydroxyl -OH radical, carboxyl radical or halogen radical such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and its tautomers such as

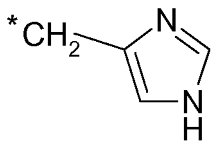

with * representing the part linked to the rest of the molecule; the dicarbonyl compounds corresponding to formula **(I),** and/or derivatives thereof and/or hydrates thereof and/or salts thereof being present in the composition in an amount ranging from 5 to 15 % in weight of the total weight of the composition;

ii) one or more acids different from the compound(s) i) as defined previously; and

iii) one or more alkalinizing agents chosen from i) aqueous ammonia, ii) alkali metal or alkaline-earth metal carbonates or hydrogen carbonates, such as sodium carbonates or hydrogen carbonates or potassium carbonates or hydrogen carbonates, iii) alkali metal or alkaline-earth metal phosphates or (di)hydrogen phosphates, iv) alkali metal or alkaline-earth metal hydroxides, such as sodium or potassium hydroxides, or mixtures thereof, v) alkanolamines, such as monoethanolamine or trihydroxyethylamine, vi) oxyethylenated and/or oxypropylenated ethylenediamines, vii) amino acids and viii) the compounds of formula (**II**) below:

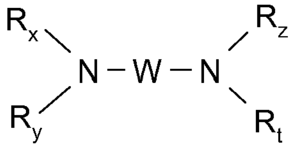
**(II)**

in which formula (**II**) **W** is a divalent $C_1$-$C_6$ alkylene radical optionally substituted with one or more hydroxyl groups or a $C_1$-$C_6$ alkyl radical, and/or optionally interrupted with one or more heteroatoms such as O, or $NR_u$; $R_x$, $R_y$, $R_z$, $R_t$ and $R_u$, which may be identical or different, represent a hydrogen atom or a $C_1$-$C_6$ alkyl, $C_1$-$C_6$ hydroxyalkyl or $C_1$-$C_6$ aminoalkyl radical, the said dicarbonyl compound(s) corresponding to formula (I) being different from pyruvic acid, a derivative thereof and hydrates thereof or salts thereof - the derivatives being chosen from esters of the carboxyl group(s), amides of the carboxyl group(s), and (thio)acetals and hemi(thio)acetals of the carbonyl function(s) of the pyruvic acid in free form or in the form of hydrates.

A subject of the invention is also a process for straightening keratin fibres, in particular the hair, using a composition comprising i) one or more dicarbonyl compounds corresponding to formula **(I)** and/or hydrates thereof and/or salts thereof as defined previously, the amount of dicarbonyl compounds of formula **(I)** and/or hydrates thereof and/or salts in a composition containing them ranging from 3 to 15% in weight of the total weight of the composition; ii) one or more acids different from the compound(s) i) as defined previously and, iii) one or more alkalinizing agents.

According to one particular embodiment of the invention, the composition comprising i), ii) and optionally iii) has an acidic pH, preferably greater than or equal to 1 and less than 7 and more particularly ranging from 1 to 4, better still from 1 to 3 and even better still from 1.7 to 3.

The process for straightening keratin fibres, in particular the hair, comprises the application to said fibres of the composition of the invention, followed by a step of straightening by means of a straightening iron at a temperature of at least 150°C, preferably inclusively between 150 and 250°C. The process is as defined in the appended claims.

The cosmetic composition of the invention is capable of being obtained by mixing at least 2 compositions, one comprising one or more dicarbonyl compounds corresponding to formula **(I)** and/or derivatives thereof and/or hydrates thereof and/or salts thereof as described hereinafter, the other comprising one or more acids different from the dicarbonyl compound(s) i) as defined previously, one or more alkalinizing agents being optionally present in either of the compositions or both.

In the present invention, the dicarbonyl compounds of formula **(I)** or derivatives thereof may be in free form, but also in hydrate forms thereof or in the form of salts thereof, preferably in free form or in the form of hydrates.

The *"derivatives"* of the dicarbonyl compounds of formula (**I**) are esters of the carboxyl group(s), amides of the carboxyl group(s), and (thio)acetals and hemi(thio)acetals of the carbonyl function(s) of the compounds of formula **(I),** in free form or optionally in the form of salts or of hydrates, preferably in free form or in the form of hydrates.

The composition of the invention is stable. The composition of the invention, and the process for treating keratin fibres using ingredients i), ii) and optionally iii) as defined previously allow good straightening of keratin fibres while limiting damage to these keratin fibres, even when the application of the composition(s) is followed by heat treatment, in particular by means of a hair-straightening iron, and have an appreciated working quality, in particular without excessive vaporization of the composition at the time of straightening. The composition and the process for treating keratin fibres

according to the invention also make it possible to limit the change in the colour of the fibres and also the problems of breaking of said fibres such as the hair. The composition and the process of the invention will also improve the physical properties of the hair, by reducing the frizziness effect in a long-lasting manner.

In that which follows, the expression *"at least one"* is equivalent to the expression *"one or more"*.

Preferably, the composition according to the invention comprises neither a colouring agent nor a reducing agent.

The term *"colouring agents"* is intended to mean, according to the present invention, agents for colouring keratin fibres such as direct dyes, pigments or oxidation dye precursors (bases and couplers). If they are present, their content does not exceed 0.001% by weight relative to the total weight of the composition. Indeed, at such a content, only the composition would be dyed, i.e. no dyeing effect would be observed on the keratin fibres.

It should be remembered that oxidation dye precursors, oxidation bases and couplers are colourless or only slightly coloured compounds which, by a condensation reaction in the presence of an oxidizing agent, give a coloured entity. With regard to direct dyes, these compounds are coloured and exhibit a degree of affinity for keratin fibres.

The term *"reducing agent"* is intended to mean, according to the present invention, an agent capable of reducing the disulfide bonds of the hair, such as the compounds chosen from thiols, alkali metal sulfites, hydrides or phosphines.

Preferably, the dicarbonyl compound(s) corresponding to formula **(I)** and/or derivatives thereof and/or hydrates thereof and/or salts thereof are chosen from the dicarbonyl compounds corresponding to formula **(I)** in which R represents i) a hydrogen atom or ii) a linear or branched $C_1$-$C_6$ alkyl group optionally substituted with a carboxyl group.

More preferably, they are chosen from glyoxylic acid and pyruvic acid, a derivative thereof and hydrates thereof or salts thereof and more preferentially from glyoxylic acid, derivatives thereof and the hydrate form thereof.

As glyoxylic acid derivatives, mention may be made of glyoxylic acid esters, glyoxylic acid amides, glyoxylic acid (thio)acetals and hemi(thio)acetals, and glyoxylic acid ester (thio)acetals and hemi(thio)acetals.

The esters and amides can be synthesized, using conventional esterification or amidation processes, from the corresponding acids well known to those skilled in the art.

Preferentially, the dicarbonyl compound(s) of formula **(I)** of the invention are chosen from glyoxylic acid and derivatives thereof and the hydrate forms of these compounds.

Mention may first of all be made of glyoxylic acid and also the hydrate form thereof $(HO)_2CH-C(O)-OH$, such as, for example, the glyoxylic acid in aqueous solution at 50% sold by the company Merck.

The glyoxylic acid esters are, for example, obtained from glyoxylic acid and a mono- or polyalcohol.

The term "*mono- or polyalcohol*" is intended to mean an organic compound comprising a hydroxyl group (monoalcohol) or at least two hydroxyl groups (polyalcohol or polyol), it being possible for said hydroxylated organic compound to be aliphatic, acyclic, linear or branched, or (hetero)cyclic, such as sugars (mono- or polysaccharides) or sugar alcohols.

More particularly, the polyalcohol comprises from 2 to 100 hydroxyl groups, and preferentially from 2 to 20 hydroxyl groups, even more preferentially from 2 to 10 hydroxyl groups and better still 2 or 3 hydroxyl groups.

Preferably, the mono- or polyalcohol is chosen from methanol, ethanol, propanol, isopropanol, butanol, hexanol, vethylene glycol, glycerol, dihydroxyacetone, glucose, sorbitol and menthol.

By way of esters, mention may particular be made of methyl glyoxylate, ethyl glyoxylate, glyceryl glyoxylate, dihydroxyacetone glyoxylate, glyceryl diglyoxylate or triglyoxylate, sorbitol mono-, di- or triglyoxylate, glucose mono-, di- or triglyoxylate, menthyl glyoxylate, and the acetals, hemiacetals and hydrates thereof.

The glyoxylic acid amides are, for example, obtained from glyoxylic acid and an organic mono- or polyamine.

The term *"mono- or polyamine"* is intended to mean an organic compound comprising an amino(monoamine) group or at least two (and preferably from 2 to 100, better still from 2 to 20) amino groups, it is possible for said organic compound to be aliphatic, acyclic, linear or branched or (hetero)cyclic.

The term *"amino"* group is intended to mean a primary amine group $-NH_2$ or a secondary amine group >NH.

Preferably, the mono- or polyamine is aliphatic.

This amine is preferably chosen from methylamine, ethylamine, propylamine, isopropylamine, butylamine, hexylamine, monoethanolamine, monopropanolamine, propane-1,2,3-triamine and diaminoacetone.

Mention may in particular be made of glyoxylic acid *N*-beta-hydroxyethylamide and glyoxylic acid *N*-gamma-hydroxypropylamide and the acetals, hemiacetals and hydrates thereof.

The glyoxylic acid (thio)acetals and hemi(thio)acetals may, for example, be obtained from the reaction of alcohols, for the acetals or hemiacetals, or of thiols, for the thioacetals or hemithioacetals, with blocked forms of glyoxylic acid, followed by hydrolysis. The alcohols can be the same as those mentioned for the esters. The thiols may be equivalents (referred to as mono- or polythiols) to the mono- or polyalcohols mentioned above, except for the fact that the hydroxyl function(s) of said mono- or polyalcohols is (are) replaced with one or more thiol functions SH of the mono- or polythiols. The acetals or thioacetals can also be cyclic (thio)acetals.

Mention may in particular be made of dimethoxyacetic acid, diethoxyacetic acid, 1,3-dioxane-2-carboxylic acid and 1,3-dioxolane-2-carboxylic acid.

The salts may be salts derived from the interaction of the compounds of formula **(I)** with acids or bases, it being possible for the acids or bases to be of organic or inorganic nature.

Preferably, the salts are salts derived from the interaction of the compounds of formula **(I)** with bases. Mention will in particular be made of the salts of alkali metals or alkaline-earth metals and in particular the sodium salts.

According to one embodiment, the composition of the invention comprises from 5% to 15% of one or more dicarbonyl compounds corresponding to formula **(I)** and/or of a derivative thereof and/or of hydrate forms thereof and/or salts thereof, preferably from 5% to 10% by weight of the total weight of the composition.

The composition according to the invention also comprises ii) one or more acids, the acid(s) being different from the compound(s) i) as defined previously.

For the purpose of the invention, the term "acid" is intended to mean an acidifying agent capable, via its presence at 1% by weight, of reducing, at 25°C, the pH of pure water or of an aqueous-ethanolic solution containing 30% by weight of ethanol by at least 0.01 unit.

According to one particular embodiment of the invention, the acid(s) ii) of the invention is (are) chosen from the following organic or inorganic acids or mixtures thereof:

- ◦ hydrochloric acid, sulfuric acid, phosphoric acid;
- ◦ sulfonic acids $\mathbf{R_a}$**-S(O)$_2$-OH,** phosphonic acids $\mathbf{R_a}$**-P(O)(OH)$_2$,** with $R_a$ representing an optionally substituted $C_1$-$C_8$ alkyl, optionally substituted (hetero)aryl or optionally substituted (hetero)aryl($C_1$-$C_8$)alkyl group;
- ◦ aromatic or non-aromatic carboxylic acids comprising at least one carboxyl function -C(O)-OH chosen from:

  $C_2$-$C_8$ monoacids corresponding to the formula $\mathbf{R_b}$**-C(O)-OH** in which the $\mathbf{R_b}$ radical represents a $C_2$-$C_8$ alkyl, (hetero)aryl or (hetero)aryl($C_2$-$C_8$)alkyl group, the alkyl part being linear or branched, the alkyl and/or (hetero)aryl part being optionally substituted, preferably with one or more hydroxyl groups, one of the hydroxyl groups preferably being separated from the carboxyl function -C(O)-OH by one or two carbon atoms; among these carboxylic acids, mention will preferentially be made of glycolic acid, lactic acid, benzoic acid and salicylic acid;

  $C_2$-$C_{30}$ diacids corresponding to the formula **HO-C(O)-**$\mathbf{R_c}$**-C(O)-OH** in which the $\mathbf{R_c}$ radical represents:

  a) a single covlent bond σ;
  b) a saturated or unsaturated, acyclic, linear or branched, divalent $C_1$-$C_{28}$, in particular $C_1$-$C_{10}$, hydrocarbon-based group, which is optionally substituted, preferably with one or more hydroxyl groups, the divalent hydrocarbon-based group preferably being a $C_1$-$C_8$ alkylene group which is optionally substituted with one or more hydroxyl groups or a ($C_2$-$C_6$)alkenylene group which is optionally substituted with one or more hydroxyl groups;
  c) a (hetero)arylene group which is optionally substituted, preferably with one or more hydroxyl groups and which is preferably an arylene group such as phenylene;
  d) a (hetero)cycloalkylene group which is optionally substituted, preferably with one or more hydroxyl groups and which is preferably a cycloalkylene group such as cyclohexylene;
  e) or a divalent group resulting from the association of radicals derived from the groups defined in b), c) and/or d), such as: -(hetero)aryl($C_1$-$C_{10}$)alkyl-; -($C_1$-$C_{10}$)alkyl(hetero)aryl($C_1$-$C_{10}$)alkyl-; - (hetero)aryl($C_1$-$C_{10}$)alkyl(hetero)aryl-; or -(hetero)cycloalkyl($C_1$-$C_{10}$)alkyl-; and more preferentially -aryl($C_1$-$C_6$)alkyl- such as - phenyl($C_1$-$C_6$)alkyl-;
  particularly, the diacids are chosen from those in which $\mathbf{R_c}$ represents a), b) or c); mention will more particularly be made of oxalic acid, malonic acid, hydroxymalonic acid, succinic acid, malic acid, tartaric acid, maleic acid, fumaric acid, itaconic acid, glutaric acid, adipic acid, azelaic acid and sebacic acid, phthalic acid, isophthalic acid and terephthalic acid;

  polyacids corresponding to the formula $\mathbf{R_d[C(O)\text{-}OH]_x}$ with **x** representing an integer greater than or equal to 3, preferably x ranging from 3 to 6, more particularly from 3 to 4 and in particular such that x is equal to 3; and $\mathbf{R_d}$ represents a polyvalent group chosen from:

  1) a saturated or unsaturated, acyclic, linear or branched, polyvalent $C_1$-$C_{28}$, in particular $C_2$-$C_{20}$, hydrocarbon-based group, which is optionally substituted with one or more groups, preferably hydroxyl groups, the hydrocarbon-based group preferably being a trivalent $C_2$-$C_8$ group which is optionally substituted with one or more hydroxyl groups;
  2) a polyvalent (hetero)aryl group which is optionally substituted, preferably with one or more hydroxyl groups, which is preferably an at least trivalent aryl group such as phenyl;
  3) a polyvalent (hetero)cycloalkyl group which is optionally substituted, preferably with one or more hydroxyl groups, which is preferably a cycloalkyl group such as cyclohexyl;
  4) or a polyvalent group resulting from the association of radicals derived from the groups defined in 1), 2) and/or 3), such as: (hetero)aryl($C_1$-$C_{10}$)alkyl; ($C_1$-$C_{10}$)alkyl(hetero)aryl($C_1$-$C_{10}$)alkyl; (hetero)ar-

yl($C_1$-$C_{10}$)alkyl(hetero)aryl; or (hetero)cycloalkyl($C_1$-$C_{10}$)alkyl; and more preferentially aryl($C_1$-$C_6$)alkyl such as phenyl($C_1$-$C_6$)alkyl;

more particularly, the polyacids being chosen from the triacids derived from groups defined in 1), in particular of $C_5$-$C_{20}$, among which mention may be made of citric acid;
◦ aromatic or non-aromatic sulfocarboxylic acids comprising at least one carboxyl function -C(O)-OH and at least one sulfonic function -$S(O)_2$-OH, such as **$[HO\text{-}C(O)]_y\text{-}R_d\text{-}[S(O)_2\text{-}OH]_z$** with **$R_d$** as defined previously for the polyacids; **y** and **z** being integers greater than or equal to 1, the sum **y + z** preferably being greater than or equal to 2 such as equal to 3;
the sulfocarboxylic acids preferably being of $C_2$-$C_{10}$, and the sulfonic acid group being separated from the carboxylic acid group(s) by a polyvalent ($C_1$-$C_6$)alkyl or aryl($C_1$-$C_6$)alkyl chain, the alkyl part of which is linear or branched, optionally substituted with a hydroxyl group. Mention may in particular be made of sulfosuccinic acid, para-sulfobenzoic acid and 4-sulfosalicylic acid;
◦ aromatic or non-aromatic phosphocarboxylic acids comprising at least one carboxyl function -C(O)-OH and at least one phosphonic function -$P(O)(OH)_2$, such as **$[HO\text{-}C(O)]_y\text{-}R_d\text{-}[P(O)(OH)_2]_z$** with **$R_d$** as defined previously for the polyacids; y and z being integers greater than or equal to 1, the sum y + z preferably being greater than or equal to 2 such as equal to 3; the phosphocarboxylic acids preferably being of $C_2$-$C_{10}$, and the phosphonic acid group being separated from the carboxylic acid group(s) by a polyvalent ($C_1$-$C_6$)alkyl or aryl($C_1$-$C_6$)alkyl group, the alkyl part of which is linear or branched and optionally substituted with a hydroxyl group. Mention may in particular be made of phosphoglycolic acid.

Unless otherwise mentioned previously:

*When the "(hetero)*aryl*" radicals or the *"(hetero)aryl"* part of a radical are optionally substituted, said radicals may then be substituted on a carbon atom, with an atom or group chosen from: i) $C_1$-$C_{16}$, preferably $C_1$-$C_8$ alkyl, optionally substituted with one or more radicals chosen from the following radicals: hydroxyl, $C_1$-$C_2$ alkoxy, (poly)-hydroxy($C_2$-$C_4$)alkoxy, acylamino, amino substituted with two identical or different $C_1$-$C_4$ alkyl radicals, optionally bearing at least one hydroxyl group, or the two radicals possibly forming, with the nitrogen atom to which they are attached, a saturated or unsaturated, optionally substituted heterocycle comprising from 5 to 7 ring members, preferably 5 or 6 ring members, optionally comprising another heteroatom which is identical to or different from nitrogen; ii) halogen; iii) hydroxyl; iv) $C_1$-$C_2$ alkoxy; v) (poly)hydroxy($C_2$-$C_4$)alkoxy; vi) amino; vii) 5- or 6-membered heterocycloalkyl; viii) optionally cationic 5- or 6-membered heteroaryl, preferentially imidazolium, and optionally substituted with a ($C_1$-$C_4$) alkyl radical, preferentially methyl; ix) amino substituted with one or two identical or different $C_1$-$C_6$ alkyl radicals optionally bearing at least one hydroxyl group, amino optionally substituted with one or two optionally substituted $C_1$-$C_3$ alkyl radicals; x) acylamino (-NR-C(O)-R') in which the R radical is a hydrogen atom, or a $C_1$-$C_4$ alkyl radical optionally bearing at least one hydroxyl group and the R' radical is a $C_1$-$C_2$ alkyl radical; xi) carbamoyl ($(R)_2$N-C(O)-) in which the R radicals, which may be identical or different, represent a hydrogen atom, or a $C_1$-$C_4$ alkyl radical optionally bearing at least one hydroxyl group; xii) alkylsulfonylamino (R'-$S(O)_2$-N(R)-) in which the R radical represents a hydrogen atom, or a $C_1$-$C_4$ alkyl radical optionally bearing at least one hydroxyl group and the R' radical represents a $C_1$-$C_4$ alkyl radical, a phenyl radical; xiii) aminosulfonyl ($(R)_2$N-$S(O)_2$-) in which the R radicals, which may be identical or different, represent a hydrogen atom, or a $C_1$-$C_4$ alkyl radical optionally bearing at least one hydroxyl group; xiv) carboxyl in acid or salified form (preferably salified with an alkali metal or an ammonium, which may or may not be substituted); xv) cyano; xvi) nitro or nitroso; xvii) polyhaloalkyl, preferentially trifluoromethyl;
the *"(hetero)cyclic"* or *"(hetero)cycloakyl"* radicals, when they are optionally substituted, may be substituted with at least one atom or group chosen from: i) hydroxyl; ii) $C_1$-$C_4$ alkoxy, (poly)hydroxy($C_2$-$C_4$)alkoxy; iii) $C_1$-$C_4$ alkyl; iv) alkylcarbonylamino (R-C(O)-N(R')-) in which the R' radical is a hydrogen atom, or a $C_1$-$C_4$ alkyl radical optionally bearing at least one hydroxyl group and the R radical is a $C_1$-$C_2$ alkyl radical or amino optionally substituted with one or two identical or different $C_1$-$C_4$ alkyl groups which are themselves optionally bearing at least one hydroxyl group, it been possible for said alkyl radicals to form, with the nitrogen atom to which they are attached, a saturated or unsaturated, optionally substituted heterocycle comprising from 5 to 7 ring members, optionally comprising at least one other heteroatom identical to or different from nitrogen; v) alkylcarbonyloxy (R-C(O)-O-) in which the R radical is a $C_1$-$C_4$ alkyl radical or an amino group optionally substituted with one or two identical or different $C_1$-$C_4$ alkyl groups which are themselves optionally bearing at least one hydroxyl group, it being possible for said alkyl radicals to form, with the nitrogen atom to which they are attached, a saturated or unsaturated, optionally substituted heterocycle comprising from 5 to 7 ring members, optionally comprising at least one other heteroatom identical to or different from nitrogen; vi) alkoxycarbonyl (R-G-C(O)-) in which the R radical is a $C_1$-$C_4$ alkoxy radical, G is an oxygen atom, or an amino group optionally substituted with a $C_1$-$C_4$ alkyl group which is itself optionally bearing at

least one hydroxyl group, it being possible for said alkyl radical to form, with the nitrogen atom to which it is attached, a saturated or unsaturated, optionally substituted heterocycle comprising from 5 to 7 ring members, optionally comprising at least one other heteroatom identical to or different from nitrogen;
the "*(hetero)cyclic*" or "*(hetero)cycloalkyl*" radicals, or a non-aromatic part of a (hetero)aryl radical, when they are optionally substituted, may also be substituted with one or more oxo groups;
a hydrocarbon-based chain is *"unsaturated"* when it comprises one or more double bonds and/or one or more triple bonds;
an "*aryl*" radical represents a fused or non-fused, monocyclic or polycyclic carbon-based group comprising from 6 to 22 carbon atoms, and in which at least one ring is aromatic; preferentially, the aryl radical is a phenyl, biphenyl, naphthyl, indenyl, anthracenyl or tetrahydronaphthyl;
a "*heteroaryl radical*" represents an optionally cationic, fused or non-fused, monocyclic or polycyclic group comprising from 5 to 22 ring members, from 1 to 6 heteroatoms chosen from nitrogen, oxygen, sulfur and selenium, at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthylyl and the ammonium salt thereof;
a *"heterocyclic radical"* or *"heterocycloalkyl radical"* is a fused or non-fused, monocyclic or polycyclic radical comprising from 5 to 22 ring members, comprising from 1 to 6 heteroatoms chosen from a nitrogen, oxygen, sulfur and selenium atom, which can contain one or two unsaturations but is non-aromatic, such as morpholino, pyperidino, pyperazino, tetrahydrofuryl or pyrrolidyl;
a *"cycloalkyl radical"* is a fused or non-fused, monocyclic or polycyclic hydrocarbon-based radical comprising from 5 to 22 ring members, which can contain one or two unsaturations but is non-aromatic, such as cyclohexyl or cyclobutyl;
an *"alkyl radical"* is a linear or branched $C_1$-$C_{20}$, preferably $C_1$-$C_8$, hydrocarbon-based radical, such as methyl or ethyl;
an *"alkenylene radical"* is an unsaturated hydrocarbon-based divalent radical as defined previously, which can contain from 1 to 4 conjugated or unconjugated double bonds -C=C-; or $-C(=CH_2)-$; the alkenylene group particularly contains 1 or 2 unsaturation(s);
the expression *"optionally substituted"* assigned to the *"alkyl", "alkylene"* or *"alkenylene"* radical or to "a *hydrocarbon-based chain"* implies that said radical may be substituted with one or more radicals chosen from the following radicals: i) hydroxyl, ii) $C_1$-$C_4$ alkoxy, iii) acylamino, iv) amino optionally substituted with one or two identical or different $C_1$-$C_4$ alkyl radicals, said alkyl radicals possibly forming, with the nitrogen atom that bears them, a heterocycle comprising from 5 to 7 ring members, optionally comprising another heteroatom identical to or different from nitrogen; v) or a quaternary ammonium group - $N^+R'R''R'''$, $M^-$ for which R', R" and R"', which may be identical or different, represent a hydrogen atom or a $C_1$-$C_4$ alkyl group, or else $-N^+R'R''R'''$ forms a heteroaryl such as imidazolium optionally substituted with a $C_1$-$C_4$ alkyl group, and $M^-$ represents the counterion of the corresponding organic acid, inorganic acid or halide;
an *"alkoxy radical"* is an alkyl-oxy radical for which the alkyl radical is a linear or branched $C_1$-$C_{16}$ and preferentially $C_1$-$C_8$ hydrocarbon-based radical;
when the alkoxy group is optionally substituted, this implies that the alkyl group is optionally substituted as defined above.

According to one advantageous embodiment of the invention, the acid(s) ii) of the invention which is (are) different from the compounds as defined previously is (are) chosen from organic acids and more particularly from aromatic or non-aromatic carboxylic acids comprising at least one carboxyl function -C(O)-OH. Preferentially, the organic acids are monocarboxylic acids, in particular chosen from glycolic acid, lactic acid and benzoic acid.
According to another particular embodiment of the invention, the acid(s) ii) of the invention is (are) chosen from inorganic acids such as phosphoric acid.
According to one particular embodiment, the composition of the invention comprises a minimal content of acids ii) which are different from the compounds as defined previously greater than or equal to 1%. Preferably, the amount of acid ii) is greater than or equal to 2% by weight relative to the total weight of the composition. Even more preferentially, the content of acid(s) ii) which is (are) different from the dicarbonyl derivatives as defined previously ranges from 2% to 10% by weight relative to the total weight of the composition.
The composition according to the invention comprises also iii) one or more alkalinizing agents.
The alkalinizing agent(s) may be inorganic, organic or hybrid.
The inorganic alkalinizing agent(s) is (are) preferably chosen from aqueous ammonia, alkali metal or alkaline-earth metals carbonates or hydrogen carbonates, such as sodium carbonates or hydrogen carbonates or potassium

carbonates or hydrogen carbonates, alkali metal or alkaline-earth metal phosphates or (di)hydrogen phosphates, alkali metal or alkaline-earth metal hydroxides, such as sodium, potassium, calcium or magnesium hydroxides, or mixtures thereof.

The organic alkalinizing agent(s) are preferably chosen from organic amines with a $pK_b$ at 25°C of less than 12, preferably less than 10 and even more advantageously less than 6. It should be noted that it is the $pK_b$ corresponding to the function of highest basicity. In addition, the organic amines do not comprise an alkyl or alkenyl fatty chain comprising more than ten carbon atoms.

The organic alkalinizing agent(s) is (are) chosen, for example, from alkanolamines, oxyethylenated and/or oxypropylenated ethylenediamines, amino acids and the compounds of formula (**II**) below:

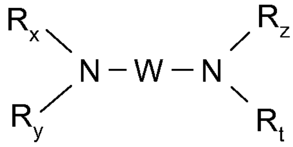

**(II)**

in which formula (**II**) **W** is a divalent $C_1$-$C_6$ alkylene radical optionally substituted with one or more hydroxyl groups or a $C_1$-$C_6$ alkyl radical, and/or optionally interrupted with one or more heteroatoms such as O, or $NR_u$; $R_x$, $R_y$, $R_z$, $R_t$ and $R_u$, which may be identical or different, represent a hydrogen atom or a $C_1$-$C_6$ alkyl, $C_1$-$C_6$ hydroxyalkyl or $C_1$-$C_6$ aminoalkyl radical.

Examples of amines of formula (**II**) that may be mentioned include 1,3-diaminopropane, 1,3-diamino-2-propanol, spermine and spermidine.

The term "alkanolamine" is intended to mean an organic amine comprising a primary, secondary or tertiary amine function, and one or more linear or branched $C_1$-$C_8$ alkyl groups bearing one or more hydroxyl radicals.

The organic amines chosen from alkanolamines such as monoalkanolamines, dialkanolamines or trialkanolamines, comprising one to three identical or different $C_1$-$C_4$ hydroxyalkyl radicals are in particular suitable for carrying out the invention.

Among compounds of this type, mention may be made of monoethanolamine (MEA), diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethylamino)methane.

More particularly, the amino acids that may be used are of natural or synthetic origin, in their L, D or racemic form, and comprise at least one acid function chosen more particularly from carboxylic acid, sulfonic acid, phosphonic acid or phosphoric acid functions. The amino acids may be in neutral or ionic form.

Advantageously, the amino acids are basic amino acids comprising an additional amine function optionally included in a ring or in a ureido function.

Such basic amino acids are preferably chosen from those corresponding to formula **(III)** below:

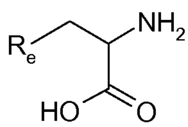

**(III)**

in which formula **(III)** $R_e$ represents a group chosen from:

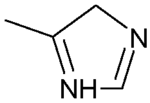 ; 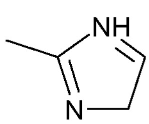 ;

$-(CH_2)_3NH_2$; $-(CH_2)_2NH_2$; $-(CH_2)_2N(H)-C(O)-NH_2$; and $-(CH_2)_2N(H)-C(NH)-NH_2$.

The compounds corresponding to formula **(III)** are histidine, lysine, arginine, ornithine or citrulline.

The organic amine may also be chosen from organic amines of heterocyclic type. Besides histidine that has already been mentioned in the amino acids, mention may in particular be made of pyridine, piperidine, imidazole, triazole, tetrazole and benzimidazole.

The organic amine can also be chosen from amino acid dipeptides. As amino acid dipeptides that may be used in the present invention, mention may be made in particular of carnosine, anserine and balenine.

The organic amine can also be chosen from compounds comprising a guanidine function. Mention may in particular be made, as amines of this type which can be used in the present invention, in addition to arginine that has already been mentioned as an amino acid, of creatine, creatinine, 1,1-dimethylguanidine, 1,1-diethylguanidine, glycocyamine, metformin, agmatine, N-amidinoalanine, 3-guanidinopropionic acid, 4-guanidinobutyric acid and 2-([amino(imino)methyl]amino)ethane-1-sulfonic acid.

Preferably, the alkalinizing agent present in the composition of the invention is an alkanolamine. Even more preferentially, the alkalinizing agent is monoethanolamine (MEA).

Mention may be made, as hybrid compounds, of the salts of the abovementioned amines with acids, such as carbonic acid or hydrochloric acid.

Use may in particular be made of guanidine carbonate or monoethanolamine hydrochloride.

The alkalinizing agents according to the invention are preferably chosen from:

- alkali metal or alkaline-earth metal hydroxides, such as sodium hydroxide, potassium hydroxide, slaked lime or magnesium hydroxide;
- aqueous ammonia;
- alkali metal or alkaline-earth metal phosphates, hydrogen phosphates or dihydrogen phosphates;
- alkali metal or alkaline-earth metal carbonates or hydrogen carbonates, such as sodium hydrogen carbonate or potassium hydrogen carbonate; and
- amines, alkylamines such as hydroxyalkylamines and quite particularly monoethanolamine (MEA), or triethanolamine.

The content of alkalinizing agent(s) optionally present is sufficient for the compositions to be at a pH preferably greater than or equal to 1 and less than 7, more particularly ranging from 1 to 4, better still from 1 to 3 and even better still from 1.7 to 3. Preferably, the alkalinizing agents introduced are present in a content ranging from 0.1% to 5% by weight of the total weight of the composition, better still from 0.3% to 1.5% by weight of the total weight of the composition.

The composition of the invention may also comprise at least one surfactant.

The surfactant(s) may be chosen from non-ionic, anionic, cationic, amphoteric or zwitterionic surfactants.

According to a particular embodiment, the composition comprises at least one amphoteric or zwitterionic surfactant.

In particular, the amphoteric or zwitterionic surfactant(s), which are preferably non-silicone, which may be used in the present invention may in particular be derivatives of optionally quaternized aliphatic secondary or tertiary amines, in which derivatives the aliphatic group is a linear or branched chain comprising from 8 to 22 carbon atoms, said amine derivatives containing at least one anionic group, for instance a carboxylate, sulfonate, sulfate, phosphate or phosphonate group.

Mention may be made in particular of $(C_8-C_{20})$alkylbetaines, $(C_8-C_{20})$alkylsulfobetaines, $(C_8-C_{20})$alkylamido$(C_3-C_8)$alkylbetaines and $(C_8-C_{20})$alkylamido$(C_6-C_8)$alkylsulfobetaines. Among the optionally quaternized secondary or tertiary aliphatic amine derivatives that may be used, as defined above, mention may also be made of the compounds of respective structures **(B1)** and **(B2)** below:

$$R_a\text{-C(O)-N(H)-CH}_2\text{-CH}_2\text{-N}^+(R_b)(R_c)\text{-CH}_2\text{-C(O)O}^-,\ M^+,X^- \qquad \textbf{(B1)}$$

in which formula **(B1):**

$R_a$ represents a $C_{10}$-$C_{30}$ alkyl or alkenyl group derived from an acid $R_a$- C(O)-OH preferably present in hydrolysed coconut oil, or a heptyl, nonyl or undecyl group;

$R_b$ represents a β-hydroxyethyl group; and

$R_c$ represents a carboxymethyl group;

$M^+$ represents a cationic counterion derived from an alkali metal or alkaline-earth metal, such as sodium, an ammonium ion or an ion derived from an organic amine, and

$X^-$ represents an organic or inorganic anionic counterion, such as that chosen from halides, acetates, phosphates, nitrates, $(C_1-C_4)$alkyl sulfates, $(C_1-C_4)$alkyl- or $(C_1-C_4)$alkylarylsulfonates, in particular methyl sulfate and ethyl sulfate; or alternatively $M^+$ and $X^-$ are absent;

$$R_{a'}\text{-C(O)-N(H)-CH}_2\text{-CH}_2\text{-N(B)(B')} \qquad \textbf{(B2)}$$

in which formula **(B2):**

B represents the group $-CH_2-CH_2-O-X'$;

B' represents the group $-(CH_2)_zY'$, with z = 1 or 2;

X' represents the group $-CH_2-C(O)OH$, $-CH_2-C(O)OZ'$, $-CH_2-CH_2-C(O)OH$, $-CH_2-CH_2-C(O)OZ'$, or a hydrogen atom;

Y' represents the group -C(O)OH, -C(O)OZ', $-CH_2CH(OH)-SO_3H$ or the group - $CH_2-CH(OH)-SO_3-Z'$;

Z' represents a cationic counterion derived from an alkali metal or alkaline-earth metal, such as sodium, an ammonium ion or an ion derived from an organic amine;

$R_{a'}$ represents a $C_{10}-C_{30}$ alkyl or alkenyl group of an acid $R_{a'}-C(O)OH$ preferably present in hydrolysed linseed oil or coconut oil, an alkyl group, in particular of $C_{17}$ and its iso form, or an unsaturated $C_{17}$ group.

The compounds of this type are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol® C2M Concentrate.

Use may also be made of compounds of formula **(B'2):**

$$R_{a''}-N(H)-CH(Y'')-(CH_2)_n-C(O)-N(H)-(CH_2)_{n'}-N(R_d)(R_e) \qquad \textbf{(B'2)}$$

in which formula **(B'2):**

Y'' represents the group -C(O)OH, -C(O)OZ'', $-CH_2-CH(OH)-SO_3H$ or the group-$CH_2-CH(OH)-SO_3-Z''$;

$R_d$ et $R_e$ represent, independently of each other, a $C_1-C_4$ alkyl or hydroxyalkyl radical;

Z'' represents a cationic counterion derived from an alkali metal or alkaline-earth metal, such as sodium, an ammonium ion or an ion derived from an organic amine;

$R_{a''}$ represents a $C_{10}-C_{30}$ alkyl or alkenyl group of an acid $R_{a''}-C(O)H$ preferably present in hydrolysed linseed oil or coconut oil; and

n and n' denote, independently of each other, an integer ranging from 1 to 3.

Among the compounds of formula (B'2), mention may be made of the compound classified in the CTFA dictionary under the name sodium diethylaminopropyl cocoaspartamide and sold by the company Chimex under the name Chimexane HB.
In accordance with a particular embodiment of the invention, the content of amphoteric or zwitterionic surfactant(s), when it (they) is (are) present, ranges from 0.05% to 30% by weight, preferably from 0.5% to 10% by weight and more preferably from 0.1% to 5% by weight, relative to the total weight of the composition.

The composition according to the invention may also comprise a cellulose-based polymer, The term *"cellulose-based"* polymer is intended to mean, according to the invention, any polysaccharide compound having in its structure sequences of glucose residues linked together via β-1,4 bonds; in addition to unsubstituted celluloses, the cellulose derivatives may be anionic, cationic, amphoteric or non-ionic. Thus, the cellulose-based polymers of the invention can be chosen from unsubstituted celluloses, including in a microcrystalline form, and cellulose ethers. Among these cellulose-based polymers, cellulose ethers, cellulose esters and cellulose ester ethers are distinguished. Among the cellulose esters are inorganic cellulose esters (cellulose nitrates, sulfates, phosphates, etc.), organic cellulose esters (cellulose monoacetates, triacetates, amidopropionates, acetate butyrates, acetate propionates and acetate trimellitates, etc.), and mixed organic/inorganic cellulose esters, such as cellulose acetate butyrate sulfates and cellulose acetate propionate sulfates. Among the cellulose ester ethers, mention may be made of hydroxypropylmethylcellulose phthalates and ethylcellulose sulfates.

The compositions according to the invention can be provided in any formulation form conventionally used and in particular in the form of an aqueous, alcoholic or aqueous/alcoholic solution or suspension or oily solution or suspension; of a solution or a dispersion of the lotion or serum type; of an emulsion, in particular having a liquid or semiliquid consistency, of the O/W, W/O or multiple type; of a suspension or emulsion having a soft consistency of (O/W) or (W/O) cream type; of an aqueous or anhydrous gel, or of any other cosmetic form.

These compositions can be packaged in pump-action sprays or in aerosol containers, in order to provide for application of the composition in the vaporized (lacquer) form or in the form of a mousse. Such packaging forms are indicated, for example, when it is desired to obtain a spray or a mousse, for the treatment of the hair. In these cases, the composition preferably comprises at least one propellant.

The compositions of the invention can be aqueous or anhydrous. They are preferably aqueous and then comprise water at a concentration ranging from 5% to 98%, better still from 5% to 90% and even better still from 10% to 90% by

weight relative to the total weight of the composition.

The composition may in particular comprise one or more organic solvents, in particular water-soluble solvents, such as $C_1$-$C_7$ alcohols; mention may in particular be made of $C_1$-$C_7$ aliphatic monoalcohols or $C_6$-$C_7$ aromatic monoalcohols $C_3$-$C_7$ polyols and $C_3$-$C_7$ polyol ethers, which may be employed alone or as a mixture with water.

The composition of the invention may also comprise at least one common cosmetic ingredient, chosen in particular from propellants; oils; solid fatty substances and in particular $C_8$-$C_{40}$ esters, $C_8$-$C_{40}$ acids; $C_8$-$C_{40}$ alcohols; sunscreens; moisturizers; antidandruff agents; antioxidants; chelating agents; nacreous agents and opacifiers; plasticizers or coalescers; fillers; silicones and in particular polydimethylsiloxanes; polymeric or non-polymeric thickeners or gelling agents other than the cellulose-based polymers already mentioned; emulsifiers; polymers, in particular conditioning or styling polymers other than those described previously; fragrances; silanes; crosslinking agents. The composition can, of course, comprise several cosmetic ingredients appearing in the above list.

Depending on their nature and the purpose of the composition, the normal cosmetic ingredients can be present in normal amounts which can be easily determined by those skilled in the art and which can be, for each ingredient, between 0.01% and 80% by weight. Those skilled in the art will take care to choose the ingredients included in the composition and the amounts thereof so that they do not harm the properties of the compositions of the present invention.

The composition according to the invention is preferably in the form of styling or care gels, care lotions or creams, conditioners, masks or sera.

The composition according to the invention can be obtained by mixing at least 2 compositions, one comprising at least ii) one acid as described previously and the other comprising i) one or more dicarbonyl compounds corresponding to formula **(I)** and/or derivatives thereof and/or hydrates thereof and/or salts thereof as described previously, one or more alkalinizing agents being present in either of the compositions or both.

A subject of the invention is also a process for straightening keratin fibres, such as the hair, using a composition comprising i) 3 to 15% of one or more dicarbonyl compounds corresponding to formula (I) and/or hydrates thereof and/or salts thereof as defined previously; and ii) one or more acids different from i) as defined previously; and, iii) one or more alkalinizing agents; with iv) a step of straightening by means of a straightening iron at a temperature of at least 150°C, preferably ranging from 150 to 250°C.

In a first variant, the process for straightening the hair comprises the application to the hair of the composition (composition A) described previously, i.e. comprising ingredients i), ii) and iii), followed iv) by a step of straightening by means of a straightening iron at a temperature of at least 150°C, preferably ranging from 150 to 250°C. Composition (A) used in the process of the invention is a defined in the process claims.

In this first variant, the process of the invention comprises the application of the composition A described previously, followed by a step of straightening keratin fibres, such as the hair, with an iron. Straightening with an iron is known from the prior art. It consists in straightening the keratin fibres, in particular the hair, with flat heating tongs, which are generally metallic. The straightening irons are generally used at a temperature ranging from 150 to 250°C.

According to one particular embodiment, the contact time of the composition A on the keratin fibres, such as the hair, ranges from 10 to 60 minutes, preferably from 20 to 40 minutes. After this or these leave-on time or times, straightening with a brush and with a hairdryer (blow-drying) is performed. The hair is then straightened with a straightening iron at a temperature ranging from 150 to 250°C and preferably ranging from 210 to 230°C.

The process of the invention may comprise the application of other hair agents as a pretreatment or post-treatment. In particular, it may comprise the application of a conditioning care product as a post-treatment.

According to another embodiment, the process for straightening keratin fibres, such as the hair, comprises a step of washing said fibres and then of drying with a hairdryer before application of the composition A. According to this particular embodiment, the steps described above are next, such as the contact times of the composition, the straightening with the straightening iron, the application of a conditioning agent and the rinsing, it being possible for all these steps to be carried out independently of one another, it been possible for blow-drying to be inserted between the contact of the composition according to the invention and the straightening with the iron. According to one particular embodiment, the straightening with the straightening iron is performed in several passes on the keratin fibres, such as the hair, in general 8 to 10 passes.

The process of the present invention is preferably performed a step of permanent reshaping at basic pH nor based on a reducing agent. According to one particular embodiment of the invention, the composition comprising ingredients i) and ii) and iii) as defined previously do not comprise a reducing agent.

The examples that follow serve to illustrate the invention.

## Examples

The following compositions were prepared:

| Compositions | 1 | 2 | 3 | 4 | 5 | 6 | 7 | 8 | 9 | 10 | 11 |
|---|---|---|---|---|---|---|---|---|---|---|---|
| Glyoxylic acid i) | 5 g | 5 g | 5 g | 5 g | 5 g | | | | | | |
| Lactic acid ii) | | | 3 g | | | | 3 g | | | | 3 g |
| Phosphoricacid ii) | | | | 5 g | | | | 5 g | | | |
| Benzoic acid ii) | | | | | 2 g | | | | 2 g | | |
| Pyruvic acid i) | | | | | | | | | | 8 g | 8 g |
| Glycolic acid ii) | | 3g | 5 g | 5 g | | 3g | | | | | |
| Sodium hydroxide iii) | qs pH 2.2 | qs pH 2.2 | qs pH 2.2 | qs pH 2.2 | qs pH 2.2 | qs pH 2.2 | qs pH 2.2 | qs pH 2.2 | qs pH 2.2 | qs pH 2.2 | qs pH 2.2 |
| Water | qs 100 g | qs 100 g | qs 100 g | qs 100 g | qs 100 g | qs 100 g | qs 100 g | qs 100 g | qs 100 g | qs 100 g | qs 100 g |

Preparation of composition **1:** sodium hydroxide at 10% is added to a solution of 5 g of glyoxylic acid and of 75 g of water, with stirring and at ambient temperature, so as to achieve a pH of 2.2, and then the medium is made up to 100 g by adding water.

Preparation of compositions **2** to **5:** respectively 3 g, 3 g, 5 g and 2 g of acid according to the composition table above are added, with stirring and at ambient temperature, to a solution of 5 g of glyoxylic acid and of 45 g of water. 20 g of water are added, the pH is adjusted to 2.2 by adding sodium hydroxide at 10% and then the medium is made up to 100 g by adding water.

Preparation of compositions **6** to **9:** 3 g, 3 g, 5 g and 2 g of acid according to the composition table above are diluted in 75 g water, the pH is adjusted to 2.2 by adding sodium hydroxide at 10% and then the medium is made up to 100 g by adding water.

Preparation of composition **10:** sodium hydroxide at 10% is added to a solution of 8 g of pyruvic acid and of 75 g of water, with stirring and at ambient temperature, so as to achieve a pH of 2.2, and then the medium is made up to 100 g by adding water. Preparation of compound **11:** 3 g of lactic acid are added, with stirring and at ambient temperature, to a solution of 8 g of pyruvic acid and of 45 g of water. 20 g of water are added, the pH is adjusted to 2.2 by adding sodium hydroxide at 10% and then the medium is made up to 100 g by adding water.

<u>One-step process for treating keratin fibres:</u>

Compositions **1** to **11,** optionally shaken before use, are applied to curly hair, which may be natural or dyed, or sensitized by a prior bleaching step, at a rate of 1 g per 2 g of hair. After 15 minutes, the hair is rinsed, dried with a hairdryer (blow-drying) and then straightened by passing over it flat tongs brought to 210°C. It is subsequently shampooed to examine the remanence of the straightening effects and of modification of the mechanical and cosmetic properties of the fibres.

Compositions **2, 3, 4** and **5** make it possible to obtain straightening properties which are superior to those obtained with compositions **1** and **6** to **9.** The properties in terms of curl relaxation, protection of the natural or artificial colour, resistance of the fibres to mechanical stresses (pulling, rubbing, twisting), sheen, smooth feel and smooth look are superior with compositions **2, 3, 4** and **5.**

Likewise, composition **11** produces performance levels which are greater than those obtained with composition **1** or **10.**

<u>Two-step process for treating keratin fibres:</u>

According to a two-step process with rinsing with water, one of compositions **6** to **9** (alternatively **1** or **10**) is applied to hair, at a rate of 1 g per 2 g of hair, the compositions are left to act for 15 minutes, the hair is rinsed, sponged and dried, and composition **1** or **10** (alternatively **6** to **9**) is applied also at a rate of 1 g per 2 g of hair for 15 minutes. The hair is then dried (blow-drying) and then straightened by passing over it flat tongs brought to 210°C (10 passes on locks separated into two thicknesses).

According to another two-step process without rinsing, one of compositions **6** to **9** (alternatively **1** or **10**) is applied to hair, at a rate of 1 g per 2 g of hair, the compositions are left to act for 15 minutes, and then, without rinsing,

composition **1** or **10** (alternatively **6** to **9**) is applied at a rate of 1 g per 2 g of hair for 15 minutes. The hair is then dried (blow-drying) and then straightened by passing over it flat tongs brought to 210°C (10 passes on locks separated into two thicknesses).

As with the one-step treatment, the application of one of composition **1** or **10,** combined with the application of compositions **6** to **9** produce performance levels which are greater than those obtained with one of compositions **1** to **10** used alone, or one of compositions **6** to **9** used alone.

Comparative example :

Compositions A and B were prepared with the following compositions :

| Compositions | A (invention) (%wt/wt) | B (Comparative) (%wt/wt) |
|---|---|---|
| GLYOXYLIC ACID | 5% AM | 5% AM |
| CITRIC ACID | 3% AM | 3% AM |
| SODIUM HYDROXIDE | 1% AM | - |
| DEIONIZED WATER | Qs 100 | Qs 100 |

2.7g hair locks of type I grey hair colored with MAJIROUGE 6.66 (MAJIREL) were washed with a shampoo and blow dried. Then 2.7g of composition A was applied to one of the hair lock and 2.7g of composition B was applied to another hair lock. After a leave-on time of 20 minutes on the hair, the locks were blow-dried with a hairdryer (brushing with 15 passes of a brush) and were then straightened with a straightening iron (10 passes). The locks were then washed with a shampoo and let dried naturally (spontaneous).

We measure the color of hair locks by using a spectrocolorimeter Konica Minolta CM 2600d (illuminant D65, angle 10°, specular components included) in the L*a*b* system.

According to this system, L* indicates the lightness of the color of the hair. The chromaticity coordinates are expressed by the parameters a* and b*, a* indicating the axis of red / green shades and b* the axis of yellow / blue shades.

ΔE corresponds to the color difference between untreated colored hair and colored hair treated with composition A or B, according to the following equation:

$$\Delta E = [(L^*-L_0^*)^2 + ( (a^* - a_0^*)^2 + (b^* - b_0^*)^2 ]^{1/2}$$

where L*, a* b* correspond to the colorimetric values for treated colored hair, and $L_0^*$, $a_0^*$ $b_0^*$ correspond to the colorimetric values for untreated colored hair.

The more important is the ΔE value, the more important the difference of color between the untreated colored hair lock and straightened colored hair lock hair is.

| | L* | a* | b* | ΔE |
|---|---|---|---|---|
| Hair locks colored with Majirouge | 26,97 | 18,15 | 6,62 | ------ |
| Hair locks colored with Majirouge and treated with the straightening process using composition A (invention) | 41 | 17,37 | 16,7 | 17,29 |
| Hair locks colored with Majirouge and treated with the straightening process using composition B (comparative) | 47,47 | 14,32 | 21,3 | 25,5 |

It has been observed that the color difference was less important when using composition A of the invention.

## Claims

**1.** Cosmetic composition comprising:

i) one or more dicarbonyl compounds corresponding to formula **(I)** below, and/or derivatives thereof and/or hydrates thereof and/or salts thereof, the derivatives being chosen from esters of the carboxyl group(s), amides

of the carboxyl group(s), and (thio)acetals and hemi(thio)acetals of the carbonyl function(s) of the compounds of formula **(I),** in free form or optionally in the form of salts or of hydrates, preferably in free form or in the form of hydrates :

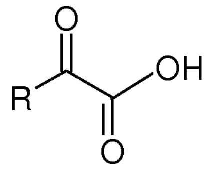

**(I)**

in which formula **(I):**
**R** represents an atom or group chosen from i) hydrogen, ii) carboxyl -C(O)-OH, iii) linear or branched $C_1$-$C_6$ alkyl which is optionally substituted, preferably with at least one hydroxyl -OH radical, carboxyl radical or halogen radical such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and its tautomers such as

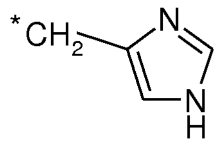

with * representing the part linked to the rest of the molecule; the dicarbonyl compounds corresponding to formula **(I),** and/or derivatives thereof and/or hydrates thereof and/or salts thereof being present in the composition in an amount ranging from 5 to 15 % in weight of the total weight of the composition;
ii) one or more acids different from the compound(s) i) as defined previously; and
iii) one or more alkalinizing agents chosen from i) aqueous ammonia, ii) alkali metal or alkaline-earth metal carbonates or hydrogen carbonates, such as sodium carbonates or hydrogen carbonates or potassium carbonates or hydrogen carbonates, iii) alkali metal or alkaline-earth metal phosphates or (di)hydrogen phosphates, iv) alkali metal or alkaline-earth metal hydroxides, such as sodium or potassium hydroxides, or mixtures thereof, v) alkanolamines, such as monoethanolamine or trihydroxyethylamine, vi) oxyethylenated and/or oxypropylenated ethylenediamines, vii) amino acids and viii) the compounds of formula **(II)** below:

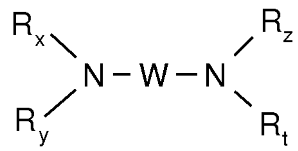

**(II)**

in which formula (**II**) **W** is a divalent $C_1$-$C_6$ alkylene radical optionally substituted with one or more hydroxyl groups or a $C_1$-$C_6$ alkyl radical, and/or optionally interrupted with one or more heteroatoms such as O, or $NR_u$; $R_x$, $R_y$, $R_z$, $R_t$ and $R_u$, which may be identical or different, represent a hydrogen atom or a $C_1$-$C_6$ alkyl, $C_1$-$C_6$ hydroxyalkyl or $C_1$-$C_6$ aminoalkyl radical;

the said dicarbonyl compound(s) corresponding to formula (I) being different from pyruvic acid, a derivative thereof and hydrates thereof or salts thereof - the derivatives being chosen from esters of the carboxyl group(s), amides of the carboxyl group(s), and (thio)acetals and hemi(thio)acetals of the carbonyl function(s) of the pyruvic acid in free form or in the form of hydrates.

**2.** Composition according to Claim 1, in which the dicarbonyl compound(s) are of formula **(I)** with R representing i) a hydrogen atom or ii) a linear or branched $C_1$-$C_6$ alkyl group optionally substituted with a carboxyl group.

**3.** Composition according to either one of the preceding claims, in which the dicarbonyl compound(s) of formula **(I)**

and/or derivatives thereof and/or hydrates thereof and/or salts thereof are chosen from glyoxylic acid, a derivative thereof, salts thereof and hydrates thereof, preferably from glyoxylic acid, a derivative thereof and the hydrate forms of these compounds.

**4.** Composition according to any one of the preceding claims, in which the dicarbonyl compound(s) of formula **(I)** and/or derivatives thereof are chosen from glyoxylic acid esters, glyoxylic acid amides, glyoxylic acid (thio)acetals and hemi(thio)acetals, and glyoxylic acid ester (thio)acetals and hemi(thio)acetals.

**5.** Composition according to Claim 3, in which the glyoxylic acid is in its hydrate form.

**6.** Composition according to any one of Claims 1 to 5, comprising from 5% to 10% by weight of one or more dicarbonyl compounds of formula **(I)** and/or derivatives thereof and/or hydrates thereof and/or salts thereof.

**7.** Composition according to any one of the preceding claims, in which the acid(s) ii) is (are) chosen from the following organic or inorganic acids, or mixtures thereof:

◦ hydrochloric acid, sulfuric acid, phosphoric acid;
◦ sulfonic acids $\mathbf{R_a\text{-}S(O)_2\text{-}OH}$, phosphonic acids $\mathbf{R_a\text{-}P(O)(OH)_2}$, with $\mathbf{R_a}$ representing an optionally substituted $C_1$-$C_8$ alkyl, optionally substituted (hetero)aryl or optionally substituted (hetero)aryl($C_1$-$C_8$)alkyl group;
◦ aromatic or non-aromatic carboxylic acids comprising at least one carboxyl function -C(O)-OH chosen from:

$C_2$-$C_8$ monoacids corresponding to the formula $\mathbf{R_b\text{-}C(O)\text{-}OH}$ in which the $\mathbf{R_b}$ radical represents a $C_2$-$C_8$alkyl, (hetero)aryl or (hetero)aryl($C_2$-$C_8$)alkyl group, the alkyl part being linear or branched, the alkyl and/or (hetero)aryl part being optionally substituted, preferably with one or more hydroxyl groups, one of the hydroxyl groups preferably being separated from the carboxyl function -C(O)-OH by one or two carbon atoms;
$C_2$-$C_{30}$ diacids corresponding to the formula $\mathbf{HO\text{-}C(O)\text{-}R_c\text{-}C(O)\text{-}OH}$ in which the $\mathbf{R_c}$ radical represents:

a) a single covalent bond $\sigma$;
b) a saturated or unsaturated, acyclic, linear or branched, divalent $C_1$-$C_{28}$, in particular $C_1$-$C_{10}$, hydrocarbon-based group, which is optionally substituted, preferably with one or more hydroxyl groups, more particularly the divalent hydrocarbon-based group preferably being a $C_1$-$C_8$ alkylene group which is optionally substituted with one or more hydroxyl groups or a ($C_2$-$C_6$)alkenylene group which is optionally substituted with one or more hydroxyl groups;
c) a (hetero)arylene group which is optionally substituted, preferably with one or more hydroxyl groups and which is preferably an arylene group such as phenylene;
d) a (hetero)cycloalkylene group which is optionally substituted, preferably with one or more hydroxyl groups and which is preferably a cycloalkylene group such as cyclohexylene;
e) or a divalent group resulting from the association of radicals derived from the groups defined in b), c) and/or d) as defined above, such as: -(hetero)aryl($C_1$-$C_{10}$)alkyl-; -($C_1$-$C_{10}$)alkyl(hetero)aryl($C_1$-$C_{10}$)alkyl-; -(hetero)aryl($C_1$-$C_{10}$)alkyl(hetero)aryl-; or -(hetero)cycloalkyl($C_1$-$C_{10}$)alkyl-; and more preferentially -aryl($C_1$-$C_6$)alkyl- such as-phenyl($C_1$-$C_6$)alkyl-;
particularly, the diacids are chosen from those in which $\mathbf{R_c}$ represents a), b) or c);

◦ polyacids corresponding to the formula $\mathbf{R_d[C(O)\text{-}OH]_x}$ with **x** representing an integer greater than or equal to 3, preferably **x** ranging from 3 to 6, more particularly from 3 to 4 and in particular such that x is equal to 3; and $\mathbf{R_d}$ represents a polyvalent group chosen from:

1) a saturated or unsaturated, acyclic, linear or branched, polyvalent $C_1$-$C_{28}$, in particular $C_2$-$C_{20}$, hydrocarbon-based group, which is optionally substituted with one or more groups, preferably hydroxyl groups, the hydrocarbon-based group preferably being a trivalent $C_2$-$C_8$ group which is optionally substituted with one or more hydroxyl groups;
2) a polyvalent (hetero)aryl group which is optionally substituted, preferably with one or more hydroxyl groups, which is preferably an at least trivalent aryl group such as phenyl;
3) a polyvalent (hetero)cycloalkyl group which is optionally substituted, preferably with one or more hydroxyl groups, which is preferably a cycloalkyl group such as cyclohexyl;
4) or a polyvalent group resulting from the association of radicals derived from the groups defined in 1), 2) and/or 3), such as: (hetero)aryl($C_1$-$C_{10}$)alkyl; ($C_1$-$C_{10}$)alkyl(hetero)aryl($C_1$-$C_{10}$)alkyl; (hetero)ar-

yl($C_1$-$C_{10}$)alkyl(hetero)aryl; or (hetero)cycloalkyl($C_1$-$C_{10}$)alkyl; and more preferentially aryl($C_1$-$C_6$)alkyl such as phenyl($C_1$-$C_6$)alkyl;

more particularly, the polyacids being chosen from the triacids derived from groups defined in 1), in particular of $C_5$-$C_{20}$;

◦ aromatic or non-aromatic sulfocarboxylic acids comprising at least one carboxyl function -C(O)-OH and at least one sulfonic function -$S(O)_2$-OH, such as **$[HO-C(O)]_y-R_d-[S(O)_2-OH]_z$** with **$R_d$** as defined previously for the polyacids; **y** and **z** being integers greater than or equal to 1, the sum y + z preferably being greater than or equal to 2 such as equal to 3; the sulfocarboxylic acids preferably being of $C_2$-$C_{10}$, and the sulfonic acid group being separated from the carboxylic acid group(s) by a polyvalent ($C_1$-$C_6$)alkyl or aryl($C_1$-$C_6$)alkyl chain, the alkyl part of which is linear or branched, optionally substituted with a hydroxyl group;

◦ aromatic or non-aromatic phosphocarboxylic acids comprising at least one carboxyl function -C(O)-OH and at least one phosphonic function -$P(O)(OH)_2$, such as **$[HO-C(O)]_y-R_d-[P(O)(OH)_2]_z$** with **$R_d$** as defined previously for the polyacids; **y** and **z** being integers greater than or equal to 1, the sum y + z preferably being greater than or equal to 2 such as equal to 3; the phosphocarboxylic acids in particular being of $C_2$-$C_{10}$, and the phosphonic acid group being separated from the carboxylic acid group(s) by a polyvalent ($C_1$-$C_6$)alkyl or aryl($C_1$-$C_6$)alkyl chain, the alkyl part of which is linear or branched and optionally substituted with a hydroxyl group.

**8.** Composition according to any one of the preceding claims, in which the acid(s) ii) is (are) chosen from organic acids.

**9.** Composition according to any one of the preceding claims, in which the acid(s) ii) is (are) chosen from aromatic or non-aromatic carboxylic acids comprising at least one carboxyl function -C(O)-OH.

**10.** Composition according to any one of the preceding claims, in which the acid(s) ii) is (are) chosen from the monocarboxylic acids as defined in Claim 7.

**11.** Composition according to any one of the preceding claims, in which the acid(s) ii) is (are) chosen from inorganic acids.

**12.** Composition according to any one of the preceding claims, in which the acid(s) ii) is (are) chosen from phosphoric acid, glycolic acid, lactic acid, benzoic acid and salicylic acid; oxalic acid, malonic acid, hydroxymalonic acid, succinic acid, malic acid, tartaric acid, maleic acid, fumaric acid, itaconic acid, glutaric acid, adipic acid, azelaic acid and sebacic acid, phthalic acid, isophthalic acid and terephthalic acid; sulfosuccinic acid, para-sulfobenzoic acid, 4-sulfosalicylic acid, phosphoglycolic acid and citric acid; preferably, the acid(s) ii) is (are) chosen from glycolic acid, lactic acid and benzoic acid.

**13.** Composition according to any one of the preceding claims, which comprises a minimal content of acids ii) as defined in any one of Claims 1 and 7 to 12 greater than or equal to 1% by weight, preferably greater than or equal to 2% by weight, the content of acid(s) ii) which is (are) different from the dicarbonyl derivatives of formula **(I)** as defined in any one of Claims 1 to 5 ranging even more preferentially from 2% to 10% by weight relative to the total weight of the composition.

**14.** Composition according to any one of the preceding claims, which has a pH greater than or equal to 1.7 and less than 7, particularly ranging from 1 to 4, more particularly ranging from 1 to 3, and preferentially ranging from 1.7 to 3.

**15.** Composition according to any one of the preceding claims, **characterized in that** it is aqueous and comprises water at a concentration particularly ranging from 5% to 98%, more particularly ranging from 5% to 90%, and preferentially ranging from 10% to 90% by weight relative to the total weight of the composition.

**16.** Process for straightening keratin fibres, such as the hair, using a composition comprising

i) one or more dicarbonyl compounds of formula **(I)** below, and/or hydrates thereof and/or salts thereof:

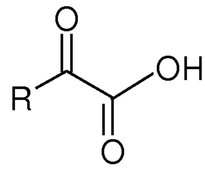

**(I)**

in which formula **(I):**
**R** represents an atom or group chosen from i) hydrogen, ii) carboxyl -C(O)-OH, iii) linear or branched $C_1$-$C_6$ alkyl which is optionally substituted, preferably with at least one hydroxyl -OH radical, carboxyl radical or halogen radical such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and its tautomers such as

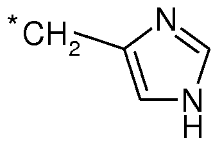

with * representing the part linked to the rest of the molecule; ;
ii) one or more acids as described in any one of Claims 1 and 7 to 13;
iii) one or more alkalinizing agents;

the amount of dicarbonyl compounds of formula **(I)** and/or hydrates thereof and/or salts in a composition containing them ranging from 3 to 15% in weight of the total weight of the composition, with iv) a step of straightening by means of a straightening iron at a temperature of at least 150°C, preferably ranging from 150 to 250 °C.

**17.** Process for straightening keratin fibres according to the preceding claim, which comprises the application to said fibres of the composition comprising:

i) one or more dicarbonyl compounds corresponding to formula **(I)** below, and/or hydrates thereof and/or salts thereof:

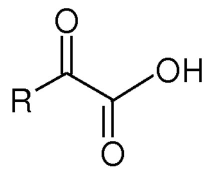

**(I)**

in which formula **(I):**
**R** represents an atom or group chosen from i) hydrogen, ii) carboxyl -C(O)-OH, iii) linear or branched $C_1$-$C_6$ alkyl which is optionally substituted, preferably with at least one hydroxyl -OH radical, carboxyl radical or halogen radical such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and its tautomers such as

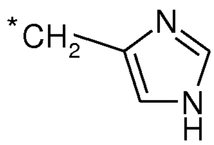

with * representing the part linked to the rest of the molecule; the dicarbonyl compounds corresponding to formula **(I),** and/or hydrates thereof and/or salts thereof being present in the composition in an amount ranging from 5 to 15 % in weight of the total weight of the composition;
ii) one or more acids different from the compound(s) i) as defined previously; and
iii) one or more alkalinizing agents chosen from i) aqueous ammonia, ii) alkali metal or alkaline-earth metal carbonates or hydrogen carbonates, such as sodium carbonates or hydrogen carbonates or potassium carbonates or hydrogen carbonates, iii) alkali metal or alkaline-earth metal phosphates or (di)hydrogen phosphates, iv) alkali metal or alkaline-earth metal hydroxides, such as sodium or potassium hydroxides, or mixtures thereof, v) alkanolamines, such as monoethanolamine or trihydroxyethylamine, vi) oxyethylenated and/or oxypropylenated ethylenediamines, vii) amino acids and viii) the compounds of formula **(II)** below:

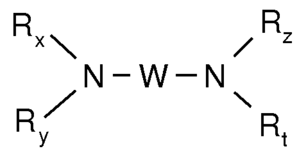

**(II)**

in which formula **(II)** **W** is a divalent $C_1$-$C_6$ alkylene radical optionally substituted with one or more hydroxyl groups or a $C_1$-$C_6$ alkyl radical, and/or optionally interrupted with one or more heteroatoms such as O, or $NR_u$; $R_x$, $R_y$, $R_z$, $R_t$ and $R_u$, which may be identical or different, represent a hydrogen atom or a $C_1$-$C_6$ alkyl, $C_1$-$C_6$ hydroxyalkyl or $C_1$-$C_6$ aminoalkyl radical,

- followed by a contact time of between 10 and 60 minutes,
- followed by a step of straightening by means of a straightening iron at a temperature of at least 150°C, preferably ranging from 150 to 250°C.

**18.** Use of a composition comprising

i) one or more dicarbonyl compounds corresponding to formula **(I)** below, and/or hydrates thereof and/or salts thereof:

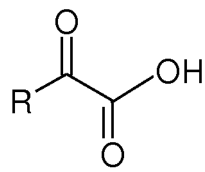

**(I)**

in which formula **(I):**
**R** represents an atom or group chosen from i) hydrogen, ii) carboxyl -C(O)-OH, iii) linear or branched $C_1$-$C_6$ alkyl which is optionally substituted, preferably with at least one hydroxyl -OH radical, carboxyl radical or halogen radical such as Br; iv) optionally substituted phenyl, v) optionally substituted benzyl, iv) and v) preferably being optionally substituted with at least one -OH or -C(O)-OH radical; vi) an indolyl radical and vii) an imidazolylmethyl radical and its tautomers such as

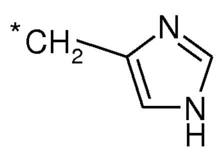

with * representing the part linked to the rest of the molecule; the dicarbonyl compounds corresponding to formula **(I),** and/or hydrates thereof and/or salts thereof being present in the composition in an amount ranging from 3 to 15 % in weight of the total weight of the composition;
ii) one or more acids different from the compound(s) i) as defined previously; and

iii) one or more alkalinizing agents, for straightening/relaxing keratin fibres, in particular the hair.

## Patentansprüche

1. Kosmetische Zusammensetzung, umfassend:

i) eine oder mehrere Dicarbonylverbindungen gemäß nachstehender Formel **(I)** und/oder Derivate davon und/oder Hydrate davon und/oder Salze davon, wobei die Derivate aus Estern der Carboxylgruppe(n), Amiden der Carboxylgruppe(n) und (Thio)acetalen und Hemi(thio)acetalen der Carbonylfunktion(en) der Verbindungen der Formel **(I)** in freier Form oder gegebenenfalls in Form von Salzen oder von Hydraten, vorzugsweise in freier Form oder in Form von Hydraten, ausgewählt sind:

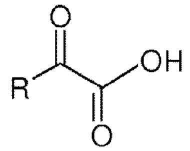

**(I)**

wobei in Formel **(I):**
**R** für ein Atom oder eine Gruppe steht, das bzw. die ausgewählt ist aus: i) Wasserstoff, ii) Carboxyl -C(O)OH, iii) geradkettigem oder verzweigtem $C_1$-$C_6$-Alkyl, das gegebenenfalls substituiert ist, vorzugsweise durch mindestens einen Hydroxylrest -OH, Carboxylrest oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie

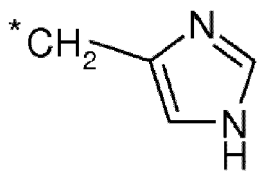 ,

wobei * für den mit dem Rest des Moleküls verknüpften Teil steht; wobei die Dicarbonylverbindungen gemäß Formel **(I)** und/oder Derivate davon und/oder Hydrate davon und/oder Salze davon in der Zusammensetzung in einer Menge im Bereich von 5 bis 15 Gew.-% des Gesamtgewichts der Zusammensetzung vorliegen;
ii) eine oder mehrere Säuren, die von der Verbindung bzw. den Verbindungen i) gemäß obiger Definition verschieden sind; und
iii) ein oder mehrere Alkalinisierungsmittel, die aus i) wässrigem Ammoniak, ii) Alkalimetall- oder Erdalkalimetallcarbonaten oder -hydrogencarbonaten, wie Natriumcarbonat oder -hydrogencarbonat oder Kaliumcarbonat oder -hydrogencarbonat, iii) Alkalimetall- oder Erdalkalimetallphosphaten oder -(di)hydrogenphosphaten, iv) Alkalimetall- oder Erdalkalimetallhydroxiden, wie Natriumhydroxid oder Kaliumhydroxid, oder Mischungen davon, v) Alkanolaminen, wie Monoethanolamin oder Trihydroxyethylamin, vi) oxyethylenierten und/oder oxypropylenierten Ethylendiaminen, vii) Aminosäuren und viii) den Verbindungen der nachstehenden Formel **(II)** ausgewählt sind:

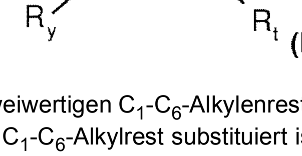 **(II)**

wobei in der Formel **(II)** **W** für einen zweiwertigen $C_1$-$C_6$-Alkylenrest steht, der gegebenenfalls durch eine oder mehrere Hydroxylgruppen oder einen $C_1$-$C_6$-Alkylrest substituiert ist und/oder gegebenenfalls durch ein oder mehrere Heteroatome wie O oder $NR_u$ unterbrochen ist; $R_x$, $R_y$, $R_z$, $R_t$ und $R_u$, die gleich oder verschieden sein

können, für ein Wasserstoffatom oder einen $C_1$-$C_6$-Alkyl-, $C_1$-$C_6$-Hydroxyalkyl- oder $C_1$-$C_6$-Aminoalkylrest stehen;

wobei die Dicarbonylverbindung(en) gemäß Formel (I) von Brenztraubensäure, einem Derivat davon und Hydraten davon oder Salzen davon verschieden ist bzw. sind - wobei die Derivate aus Estern der Carboxylgruppe(n), Amiden der Carboxylgruppe(n) und (Thio)acetalen und Hemi(thio)acetalen der Carbonylfunktion(en) der Brenztraubensäure in freier Form oder in Form von Hydraten ausgewählt sind.

2. Zusammensetzung nach Anspruch 1, wobei die Dicarbonylverbindung(en) die Formel **(I)** aufweist bzw. aufweisen, wobei R für i) ein Wasserstoffatom oder ii) eine geradkettige oder verzweigte $C_1$-$C_6$-Alkylgruppe, die gegebenenfalls durch eine Carboxylgruppe substituiert ist, steht.

3. Zusammensetzung nach einem der vorhergehenden Ansprüche, wobei die Dicarbonylverbindung(en) der Formel **(I)** und/oder Derivate davon und/oder Hydrate davon und/oder Salze davon aus Glyoxylsäure, einem Derivat davon, Salzen davon und Hydraten davon, vorzugsweise aus Glyoxylsäure, einem Derivat davon und den Hydratformen dieser Verbindungen, ausgewählt ist bzw. sind.

4. Zusammensetzung nach einem der vorhergehenden Ansprüche, wobei die Dicarbonylverbindung(en) der Formel **(I)** und/oder Derivate davon aus Glyoxylsäureestern, Glyoxylsäureamiden, Glyoxylsäure-(thio)acetalen und -hemi(thio)acetalen und Glyoxylsäureester(thio)acetalen und -hemi(thio)-acetalen ausgewählt ist bzw. sind.

5. Zusammensetzung nach Anspruch 3, wobei die Glyoxylsäure in ihrer Hydrateform vorliegt.

6. Zusammensetzung nach einem der Ansprüche 1 bis 5, umfassend 5 bis 10 Gew.-% einer oder mehrerer Dicarbonylverbindungen der Formel **(I)** und/oder Derivate davon und/oder Hydrate davon und/oder Salze davon.

7. Zusammensetzung nach einem der vorhergehenden Ansprüche, wobei die Säure(n) ii) aus den folgenden organischen oder anorganischen Säuren oder Mischungen davon ausgewählt ist (sind):

◦ Salzsäure, Schwefelsäure, Phosphorsäure;
◦ Sulfonsäuren $\mathbf{R_a\text{-}S(O)_2\text{-}OH}$, Phosphonsäuren $\mathbf{R_a\text{-}P(O)(OH)_2}$, wobei $\mathbf{R_a}$ für eine gegebenenfalls substituierte $C_1$-$C_8$-Alkyl-, gegebenenfalls substituierte (Hetero)aryl- oder gegebenenfalls substituierte (Hetero)aryl)($C_1$-$C_8$)alkylgruppe steht;
◦ aromatischen oder nichtaromatischen Carbonsäuren mit mindestens einer Carboxylfunktion -C(O)OH, ausgewählt aus:

$C_2$-$C_8$-Monosäuren gemäß der Formel $\mathbf{R_b\text{-}C(O)\text{-}OH}$, in der der $\mathbf{R_b}$-Rest für eine $C_2$-$C_8$-Alkyl-, (Hetero)aryl- oder (Hetero)aryl($C_2$-$C_8$)alkylgruppe steht, wobei der Alkylteil linear oder verzweigt ist und der Alkyl- und/oder (Hetero)arylteil gegebenenfalls substituiert ist, vorzugsweise durch eine oder mehrere Hydroxylgruppen, wobei eine der Hydroxylgruppen vorzugsweise durch ein oder zwei Kohlenstoffatome von der Carboxylfunktion -C(O)OH getrennt ist;
$C_2$-$C_{30}$-Disäuren gemäß der Formel $\mathbf{HO\text{-}C(O)\text{-}R_c\text{-}C(O)\text{-}OH}$, in der der $\mathbf{R_c}$-Rest für

a) eine kovalente Einfachbindung $\sigma$;
b) eine gesättigte oder ungesättigte, acyclische, lineare oder verzweigte, zweiwertige $C_1$-$C_{28}$- und insbesondere $C_1$-$C_{10}$-Gruppe auf Kohlenwasserstoffbasis, die gegebenenfalls substituiert ist, vorzugsweise durch eine oder mehrere Hydroxylgruppen, spezieller die zweiwertige Gruppe auf Kohlenwasserstoffbasis vorzugsweise eine $C_1$-$C_8$-Alkylengruppe, die gegebenenfalls durch eine oder mehrere Hydroxylgruppen substituiert ist, oder eine ($C_2$-$C_6$)Alkenylen-gruppe, die gegebenenfalls durch eine oder mehrere Hydroxylgruppen substituiert ist, ist;
c) eine (Hetero)arylengruppe, die gegebenenfalls substituiert ist, vorzugsweise durch eine oder mehrere Hydroxylgruppen, und die vorzugsweise eine Arylengruppe wie Phenylen ist;
d) eine (Hetero)cycloalkylengruppe, die gegebenenfalls substituiert ist, vorzugsweise durch eine oder mehrere Hydroxylgruppen, und die vorzugsweise eine Cycloalkylengruppe wie Cyclohexylen ist;
e) oder eine zweiwertige Gruppe, die sich aus der Assoziation von Resten, die sich von den in b), c) und/oder d) gemäß obiger Definition ableiten, ergeben, wie - (Hetero)aryl($C_1$-$C_{10}$)alkyl-; - ($C_1$-$C_{10}$)-Alkyl(hetero)aryl($C_1$-$C_{10}$)alkyl-; -(Hetero)aryl($C_1$-$C_{10}$)alkyl(hetero)aryl- oder - (Hetero)cycloalkyl($C_1$-$C_{10}$)alkyl-; und weiter bevorzugt -Aryl($C_1$-$C_6$)alkyl- wie -Phenyl($C_1$-$C_6$)alkyl-;

steht; insbesondere die Disäuren aus denjenigen ausgewählt sind, in denen $\mathbf{R_c}$ für a), b) oder c) steht;

◦ Polysäuren gemäß der Formel $\mathbf{R_d[C(O)\text{-}OH]_x}$, wobei **x** für eine ganze Zahl größer oder gleich 3 steht, vorzugsweise **x** im Bereich von 3 bis 6, spezieller von 3 bis 4, liegt und insbesondere derart, dass x gleich 3 ist; und $\mathbf{R_d}$ für eine mehrwertige Gruppe steht, die aus

1) einer gesättigten oder ungesättigten, acyclischen, linearen oder verzweigten, mehrwertigen $C_1$-$C_{28}$- und insbesondere $C_2$-$C_{20}$-Gruppe auf Kohlenwasserstoffbasis, die gegebenenfalls durch eine oder mehrere Gruppen, vorzugsweise Hydroxylgruppen, substituiert ist, wobei die Gruppe auf Kohlenwasserstoffbasis vorzugsweise eine dreiwertige $C_2$-$C_8$-Gruppe, die gegebenenfalls durch eine oder mehrere Hydroxylgruppen substituiert ist, ist;
2) eine mehrwertige (Hetero)arylgruppe, die gegebenenfalls substituiert ist, vorzugsweise durch eine oder mehrere Hydroxylgruppen, und die vorzugsweise eine dreiwertige Arylgruppe wie Phenyl ist;
3) eine mehrwertige (Hetero)cycloalkylgruppe, die gegebenenfalls substituiert ist, vorzugsweise durch eine oder mehrere Hydroxylgruppen, die vorzugsweise eine Cycloalkylgruppe wie Cyclohexyl ist;
4) oder eine mehrwertige Gruppe, die sich aus der Assoziation von Resten, die sich von den in 1), 2) und/oder 3) definierten Gruppen ableiten, ergeben, wie (Hetero)aryl($C_1$-$C_{10}$)alkyl; ($C_1$-$C_{10}$) - Alkyl(hetero)aryl($C_1$-$C_{10}$)alkyl; (Hetero) - aryl($C_1$-$C_{10}$)alkyl(hetero)aryl oder (Hetero)cycloalkyl($C_1$-$C_{10}$)alkyl und weiter bevorzugt Aryl($C_1$-$C_6$)alkyl wie Phenyl($C_1$-$C_6$)alkyl;

ausgewählt ist; spezieller die Polysäuren aus den Trisäuren, die sich von in 1) definierten Gruppen ableiten, insbesondere mit 5 bis 20 C-Atomen, ausgewählt sind;
◦ aromatischen oder nichtaromatischen Sulfocarbonsäuren mit mindestens einer Carboxylfunktion -C(O)-OH und mindestens einer Sulfonsäurefunktion -S(O)$_2$-OH, wie $\mathbf{[HO\text{-}C(O)]_y\text{-}R_d\text{-}[S(O)_2\text{-}OH]_z}$, wobei $\mathbf{R_d}$ wie oben für die Polysäuren definiert ist; **y** und **z** für ganze Zahlen größer oder gleich 1 stehen, wobei die Summe y + z vorzugsweise größer oder gleich 2, wie gleich 3, ist; wobei die Sulfocarbonsäuren vorzugsweise 2 bis 10 C-Atome aufweisen, und wobei die Sulfonsäuregruppe durch eine mehrwertige ($C_1$-$C_6$)Alkyl- oder Aryl ($C_1$-$C_6$) alkylkette, deren Alkylteil linear oder verzweigt und gegebenenfalls durch eine Hydroxylgruppe substituiert ist, von der Carbonsäuregruppe bzw. den Carbonsäuregruppe getrennt ist;
◦ aromatischen oder nichtaromatischen Phosphocarbonsäuren mit mindestens einer Carboxylfunktion -C(O)-OH und mindestens einer Phosphonsäurefunktion -P(O)(OH)$_2$, wie $\mathbf{[HO\text{-}C(O)]_y\text{-}R_d\text{-}[P(O)(OH)_2]_z}$, wobei $\mathbf{R_d}$ wie oben für die Polysäuren definiert ist; **y** und **z** für ganze Zahlen größer oder gleich 1 stehen, wobei die Summe y + z vorzugsweise größer oder gleich 2, wie gleich 3, ist; wobei die Phosphocarbonsäuren vorzugsweise 2 bis 10 C-Atome aufweisen, und wobei die Phosphonsäuregruppe durch eine mehrwertige ($C_1$-$C_6$) Alkyl- oder Aryl ($C_1$-$C_6$) alkylkette, deren Alkylteil linear oder verzweigt und gegebenenfalls durch eine Hydroxylgruppe substituiert ist, von der Carbonsäuregruppe bzw. den Carbonsäuregruppe getrennt ist.

**8.** Zusammensetzung nach einem der vorhergehenden Ansprüche, wobei die Säure(n) ii) aus organischen Säuren ausgewählt ist (sind).

**9.** Zusammensetzung nach einem der vorhergehenden Ansprüche, wobei die Säure(n) ii) aus aromatischen oder nichtaromatischen Carbonsäuren mit mindestens einer Carboxylfunktion -C(O)-OH ausgewählt ist (sind).

**10.** Zusammensetzung nach einem der vorhergehenden Ansprüche, wobei die Säure(n) ii) aus den Monocarbonsäuren gemäß Anspruch 7 ausgewählt ist (sind).

**11.** Zusammensetzung nach einem der vorhergehenden Ansprüche, wobei die Säure(n) ii) aus anorganischen Säuren ausgewählt ist (sind).

**12.** Zusammensetzung nach einem der vorhergehenden Ansprüche, wobei die Säure(n) ii) aus Phosphorsäure, Glykolsäure, Milchsäure, Benzoesäure und Salicylsäure; Oxalsäure, Malonsäure, Hydroxymalonsäure, Bernsteinsäure, Äpfelsäure, Weinsäure, Maleinsäure, Fumarsäure, Itaconsäure, Glutarsäure, Adipinsäure, Azelainsäure und Sebacinsäure, Phthalsäure, Isophthalsäure und Terephthalsäure; Sulfobernsteinsäure, para-Sulfobenzoesäure, 4-Sulfosalicylsäure, Phosphoglykolsäure und Citronensäure ausgewählt ist (sind); vorzugsweise die Säure (n) ii) aus Glykolsäure, Milchsäure und Benzoesäure ausgewählt ist (sind).

**13.** Zusammensetzung nach einem der vorhergehenden Ansprüche, die einen Mindestgehalt an Säuren ii) gemäß einem der Ansprüche 1 und 7 bis 12 größer oder gleich 1 Gew.-%, vorzugsweise größer oder gleich 2 Gew.-%,

umfasst, wobei der Gehalt an Säure (n) ii), die von den Carbonylderivaten der Formel **(I)** gemäß einem der Ansprüche 1 bis 5 verschieden ist (sind), noch weiter bevorzugt im Bereich von 2 bis 10 Gew.-%, bezogen auf das Gesamtgewicht der Zusammensetzung, liegt.

**14.** Zusammensetzung nach einem der vorhergehenden Ansprüche, die einen pH-Wert größer oder gleich 1,7 und weniger als 7, insbesondere im Bereich von 1 bis 4, spezieller im Bereich von 1 bis 3 und vorzugsweise im Bereich von 1,7 bis 3 aufweist.

**15.** Zusammensetzung nach einem der vorhergehenden Ansprüche, **dadurch gekennzeichnet, dass** sie wässrig ist und Wasser in einer Konzentration, die vorzugsweise im Bereich von 5 bis 98 Gew.-%, noch besser von 5 bis 90 Gew.-% und sogar noch besser von 10 bis 90 Gew.-%, bezogen auf das Gesamtgewicht der Zusammensetzung, liegt, umfasst.

**16.** Verfahren zum Glätten von Keratinfasern, wie des Haars, unter Verwendung einer Zusammensetzung, umfassend i) eine oder mehrere Dicarbonylverbindungen gemäß nachstehender Formel **(I)** und/oder Hydrate davon und/oder Salze davon:

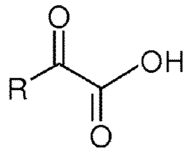

**(I)**

wobei in Formel **(I):**
**R** für ein Atom oder eine Gruppe steht, das bzw. die ausgewählt ist aus:

i) Wasserstoff, ii) Carboxyl -C(O)OH, iii) geradkettigem oder verzweigtem $C_1$-$C_6$-Alkyl, das gegebenenfalls substituiert ist, vorzugsweise durch mindestens einen Hydroxylrest -OH, Carboxylrest oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie

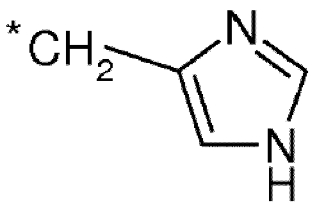

,

wobei * für den mit dem Rest des Moleküls verknüpften Teil steht;
ii) eine oder mehrere Säuren gemäß einem der Ansprüche 1 und 7 bis 13;
iii) ein oder mehrere Alkalinisierungsmittel;

wobei die Menge an Dicarbonylverbindungen der Formel (I) und/oder Hydraten davon und/oder Salzen davon in einer Zusammensetzung, die sie enthält, im Bereich von 3 bis 15 Gew.-% des Gesamtgewichts der Zusammensetzung liegt, mit iv) einem Glättungsschritt unter Verwendung eines Glätteisens bei einer Temperatur von mindestens 150 °C, vorzugsweise im Bereich von 150 bis 250 °C.

**17.** Verfahren zum Glätten von Keratinfasern nach dem vorhergehenden Anspruch, umfassend das Aufbringen der Zusammensetzung, umfassend:

i) eine oder mehrere Dicarbonylverbindungen gemäß nachstehender Formel **(I)** und/oder Hydrate davon und/oder Salze davon:

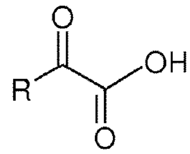

**(I)**

wobei in Formel **(I):**
**R** für ein Atom oder eine Gruppe steht, das bzw. die ausgewählt ist aus: i) Wasserstoff, ii) Carboxyl -C(O)OH, iii) geradkettigem oder verzweigtem $C_1$-$C_6$-Alkyl, das gegebenenfalls substituiert ist, vorzugsweise durch mindestens einen Hydroxylrest -OH, Carboxylrest oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie

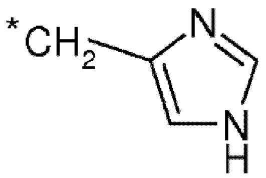
,

wobei * für den mit dem Rest des Moleküls verknüpften Teil steht; wobei die Dicarbonylverbindungen gemäß Formel **(I)** und/oder Hydrate davon und/oder Salze davon in der Zusammensetzung in einer Menge im Bereich von 5 bis 15 Gew.-% des Gesamtgewichts der Zusammensetzung vorliegen;
ii) eine oder mehrere Säuren, die von der Verbindung bzw. den Verbindungen i) gemäß obiger Definition verschieden sind; und
iii) ein oder mehrere Alkalinisierungsmittel, die aus i) wässrigem Ammoniak, ii) Alkalimetall- oder Erdalkalimetallcarbonaten oder -hydrogencarbonaten, wie Natriumcarbonat oder -hydrogencarbonat oder Kaliumcarbonat oder -hydrogencarbonat, iii) Alkalimetall- oder Erdalkalimetallphosphaten oder -(di)hydrogenphosphaten, iv) Alkalimetall- oder Erdalkalimetallhydroxiden, wie Natriumhydroxid oder Kaliumhydroxid, oder Mischungen davon, v) Alkanolaminen, wie Monoethanolamin oder Trihydroxyethylamin, vi) oxyethylenierten und/oder oxypropylenierten Ethylendiaminen, vii) Aminosäuren und viii) den Verbindungen der nachstehenden Formel **(II)** ausgewählt sind:

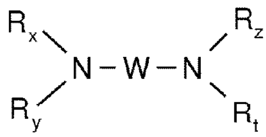
**(II)**

wobei in der Formel **(II)** **W** für einen zweiwertigen $C_1$-$C_6$-Alkylenrest steht, der gegebenenfalls durch eine oder mehrere Hydroxylgruppen oder einen $C_1$-$C_6$-Alkylrest substituiert ist und/oder gegebenenfalls durch ein oder mehrere Heteroatome wie O oder $NR_u$ unterbrochen ist; $R_x$, $R_y$, $R_z$, $R_t$ und $R_u$, die gleich oder verschieden sein können, für ein Wasserstoffatom oder einen $C_1$-$C_6$-Alkyl-, $C_1$-$C_6$-Hydroxyalkyl- oder $C_1$-$C_6$-Aminoalkylrest stehen;

auf die Fasern;

- gefolgt von einer Kontaktzeit von 10 bis 60 Minuten,
- gefolgt von einem Glättungsschritt unter Verwendung eines Glätteisens bei einer Temperatur von mindestens 150 °C, vorzugsweise im Bereich von 150 bis 250 °C.

**18.** Verwendung einer Zusammensetzung, umfassend:

i) eine oder mehrere Dicarbonylverbindungen gemäß nachstehender Formel **(I)** und/oder Hydrate davon

und/oder Salze davon:

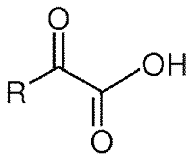

**(I)**

wobei in Formel **(I):**
**R** für ein Atom oder eine Gruppe steht, das bzw. die ausgewählt ist aus: i) Wasserstoff, ii) Carboxyl -C(O)OH, iii) geradkettigem oder verzweigtem $C_1$-$C_6$-Alkyl, das gegebenenfalls substituiert ist, vorzugsweise durch mindestens einen Hydroxylrest -OH, Carboxylrest oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie

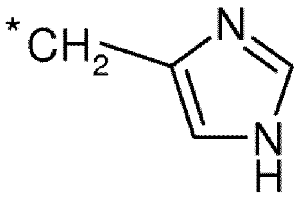,

wobei * für den mit dem Rest des Moleküls verknüpften Teil steht; wobei die Dicarbonylverbindungen gemäß Formel **(I)** und/oder Hydrate davon und/oder Salze davon in der Zusammensetzung in einer Menge im Bereich von 3 bis 15 Gew.-% des Gesamtgewichts der Zusammensetzung vorliegen;
ii) eine oder mehrere Säuren, die von der Verbindung bzw. den Verbindungen i) gemäß obiger Definition verschieden sind; und
iii) ein oder mehrere Alkalinisierungsmittel, zum Glätten/Entkräuseln von Keratinfasern, insbesondere dem Haar.

## Revendications

**1.** Composition cosmétique comprenant :

i) un ou plusieurs composés dicarbonyliques correspondant à la formule (I) ci-dessous, et/ou des dérivés de ceux-ci et/ou des hydrates de ceux-ci et/ou des sels de ceux-ci, les dérivés étant choisis parmi les esters du ou des groupes carboxyle, les amides du ou des groupes carboxyle et un ou des (thio)acétals et un ou des hémi (thio) acétals de la ou des fonctions carbonyle des composés de formule (I), sous une forme libre ou éventuellement sous la forme de sels ou d'hydrates, de préférence sous une forme libre ou sous la forme d'hydrates :

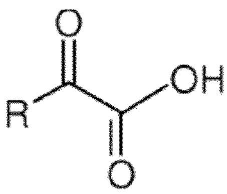

**(I)**

dans ladite formule (I) :
R représente un atome ou un groupe choisi parmi i) un hydrogène, ii) un carboxyle -C(O)-OH, iii) un alkyle en $C_1$ à $C_6$ linéaire ou ramifié qui est éventuellement substitué, de préférence par au moins un radical hydroxyle

-OH, un radical carboxyle ou un radical halogène tel que Br ; iv) un phényle éventuellement substitué, v) un benzyle éventuellement substitué, iv) et v) étant de préférence éventuellement substitués par au moins un radical -OH ou -C(O)-OH ; vi) un radical indolyle et vii) un radical imidazolylméthyle et ses tautomères tels que

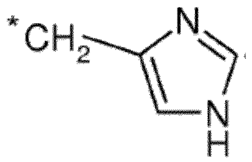

où * représente la partie liée au reste de la molécule ; les composés dicarbonyliques correspondant à la formule (I) et/ou les dérivés de ceux-ci et/ou les hydrates de ceux-ci et/ou les sels de ceux-ci étant présents dans la composition en une quantité allant de 5 à 15 % en poids du poids total de la composition ;
ii) un ou plusieurs acides différents du ou des composés i) tels que définis précédemment ; et
iii) un ou plusieurs agents alcalinisants choisis parmi i) l'ammoniaque aqueuse, ii) les carbonates ou les hydrogénocarbonates de métaux alcalins ou de métaux alcalino-terreux, tels que les carbonates ou les hydrogénocarbonates de sodium ou les carbonates ou les hydrogénocarbonates de potassium, iii) les phosphates ou les (di)hydrogénophosphates de métaux alcalins ou de métaux alcalino-terreux, iv) les hydroxydes de métaux alcalins ou de métaux alcalino-terreux, tels que les hydroxydes de sodium ou de potassium, ou des mélanges de ceux-ci, v) les alcanolamines, telles que la monoéthanolamine ou la trihydroxyéthylamine, vi) les éthylènediamines oxyéthylées et/ou oxypropylées, vii) les acides aminés et viii) les composés de formule (II) ci-dessous :

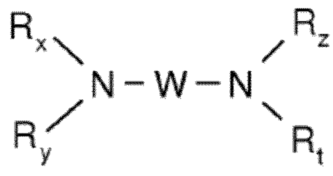
**(II)**

dans ladite formule (II) W représente un radical alkylène en $C_1$ à $C_6$ divalent éventuellement substitué par un ou plusieurs groupes hydroxyle ou un radical alkyle en $C_1$ à $C_6$, et/ou éventuellement interrompu par un ou plusieurs hétéroatomes tels que O, ou $NR_u$ ; $R_x$, $R_y$, $R_z$, $R_t$ et $R_u$, qui peuvent être identiques ou différents, représentent un atome d'hydrogène ou un radical alkyle en $C_1$ à $C_6$, hydroxyalkyle en $C_1$ à $C_6$ ou aminoalkyle en $C_1$ à $C_6$ ;

ledit ou lesdits composés dicabonyliques correspondant à la formule (I) étant différents de l'acide pyruvique, d'un dérivé de celui-ci et des hydrates de celui-ci ou des sels de celui-ci - les dérivés étant choisis parmi les esters du ou des groupes carboxyle, les amides du ou des groupes carboxyle, et un ou des (thio)acétals et un ou des hémi(thio)acétals de la ou des fonctions carbonyle de l'acide pyruvique sous une forme libre ou sous la forme d'hydrates.

**2.** Composition selon la revendication 1, dans laquelle le ou les composés dicarbonyliques sont de formule (I) où R représente i) un atome d'hydrogène ou ii) un groupe alkyle en $C_1$ à $C_6$ linéaire ou ramifié éventuellement substitué par un groupe carboxyle.

**3.** Composition selon l'une quelconque des revendications précédentes, dans laquelle le ou les composés dicarbonyliques de formule (I) et/ou les dérivés de ceux-ci et/ou les hydrates de ceux-ci et/ou les sels de ceux-ci sont choisis parmi l'acide glyoxylique, un dérivé de celui-ci, un sel de celui-ci et des hydrates de celui-ci, de préférence l'acide glycolique, un dérivé de celui-ci et les formes hydratées de ces composés.

**4.** Composition selon l'une quelconque des revendications précédentes, dans laquelle le ou les composés dicarbonyliques de formule (I) et/ou les dérivés de ceux-ci sont choisis parmi les esters de l'acide glyoxylique, les amides de l'acide glyoxylique, les (thio)acétals et les hémi(thio)acétals de l'acide glyoxylique, et les (thio)acétals et les hémi(thio)acétals d'un ester de l'acide glyoxylique.

**5.** Composition selon la revendication 3, dans laquelle l'acide glyoxylique est sous sa forme hydratée.

**6.** Composition selon l'une quelconque des revendications 1 à 5, comprenant de 5 % à 10 % en poids d'un ou plusieurs composés dicarbonyliques de formule (I) et/ou dérivés de ceux-ci et/ou hydrates de ceux-ci et/ou sels de ceux-ci.

**7.** Composition selon l'une quelconque des revendications précédentes, dans laquelle le ou les acides ii) sont choisis parmi les acides organiques ou inorganiques suivants, ou des mélanges de ceux-ci :

◦ l'acide chlorhydrique, l'acide sulfurique, l'acide phosphorique ;
◦ les acides sulfoniques $R_a$-S(O)$_2$-OH, les acides phosphoniques $R_a$-P(O)(OH)$_2$, où $R_a$ représente un groupe alkyle en $C_1$-$C_8$ éventuellement substitué, (hétéro)aryle éventuellement substitué ou (hétéro)aryl(alkyle en $C_1$ à $C_8$) éventuellement substitué ;
◦ les acides carboxyliques aromatiques ou non aromatiques comprenant au moins une fonction carboxyle -C(O)-OH choisie parmi :

les monoacides en $C_2$ à $C_8$ correspondant à la formule $R_b$-C(O)-OH dans laquelle le radical $R_b$ représente un groupe alkyle en $C_2$-$C_8$, (hétéro) aryle ou (hétéro)aryl(alkyle en $C_2$ à $C_8$), la partie alkyle étant linéaire ou ramifiée, la partie alkyle et/ou (hétéro)aryle étant éventuellement substituée, de préférence par un ou plusieurs groupes hydroxyle, l'un des groupes hydroxyle étant de préférence séparé de la fonction carboxyle -C(O)-OH par un ou deux atomes de carbone ;

les diacides en $C_2$ à $C_{30}$ correspondant à la formule HO-C(O)-$R_c$-C(O)-OH dans laquelle le radical $R_c$ représente :

a)une liaison covalente simple $\sigma$ ;
b)un groupe à base d'hydrocarbure saturé ou insaturé, acyclique, linéaire ou ramifié, divalent en $C_1$ à $C_{28}$, en particulier en $C_1$ à $C_{10}$, qui est éventuellement substitué, de préférence par un ou plusieurs groupes hydroxyle, plus particulièrement le groupe à base d'hydrocarbure divalent étant de préférence un groupe alkylène en $C_1$ à $C_8$ qui est éventuellement substitué par un ou plusieurs groupes hydroxyle ou un groupe alcénylène en $C_2$ à $C_6$ qui est éventuellement substitué par un ou plusieurs groupes hydroxyle ;
c)un groupe (hétéro)arylène qui est éventuellement substitué, de préférence par un ou plusieurs groupes hydroxyle et qui est de préférence un groupe arylène tel que le phénylène ;
d) un groupe (hétéro)cycloalkylène qui est éventuellement substitué, de préférence par un ou plusieurs groupes hydroxyle et qui est de préférence un groupe cycloalkylène tel que le cyclohexylène ;
e) ou un groupe divalent résultant de l'association de radicaux dérivés des groupes définis dans b), c) et/ou d) tels que définis ci-dessus, tels que : -(hétéro)aryl(alkyle en $C_1$ à $C_{10}$) - ; -(alkyle en $C_1$ à $C_{10}$)(hétéro)aryl(alkyle en $C_1$ à $C_{10}$) - ; - (hétéro)aryl(alkyle en $C_1$ à $C_{10}$)(hétéro)aryle- ; ou - (hétéro)cycloalkyl(alkyle en $C_1$ à $C_{10}$) - ; et plus préférentiellement -aryl(alkyle en $C_1$ à $C_6$) - tel que -phényl(alkyle en $C_1$ à $C_6$) - ;
en particulier les diacides sont choisis parmi ceux dans lesquels $R_c$ représente a), b) ou c) ;

◦ les polyacides correspondant à la formule $R_d$[C(O) - OH]$_x$ où x représente un entier supérieur ou égal à 3, de préférence x est compris dans la plage allant de 3 à 6, plus particulièrement de 3 à 4 et en particulier de sorte que x est égal à 3 ; et $R_d$ représente un groupe polyvalent choisi parmi :

1) un groupe à base d'hydrocarbure saturé ou insaturé, acyclique, linéaire ou ramifié, polyvalent en $C_1$ à $C_{28}$, en particulier en $C_2$ à $C_{20}$, qui est éventuellement substitué par un ou plusieurs groupes, de préférence par des groupes hydroxyle, le groupe à base d'hydrocarbure étant de préférence un groupe trivalent en $C_2$ à $C_8$ qui est éventuellement substitué par un ou plusieurs groupes hydroxyle ;
2) un groupe (hétéro)aryle polyvalent qui est éventuellement substitué, de préférence par un ou plusieurs groupes hydroxyle, qui est de préférence un groupe aryle au moins trivalent tel que le phényle ;
3) un groupe (hétéro)cycloalkyle polyvalent qui est éventuellement substitué, de préférence par un ou plusieurs groupes hydroxyle, qui est de préférence un groupe cycloalkyle tel que le cyclohexyle ;
4) ou un groupe polyvalent résultant de l'association de radicaux dérivés des groupes définis dans 1), 2) et/ou 3) tels que : (hétéro)aryl(alkyle en $C_1$ à $C_{10}$) ; (alkyle en $C_1$ à $C_{10}$)(hétéro)aryl(alkyle en $C_1$ à $C_{10}$) ; (hétéro)aryl(alkyle en $C_1$ à $C_{10}$)(hétéro)aryle ; ou (hétéro)cycloalkyl(alkyle en $C_1$ à $C_{10}$) ; et plus préférentiellement aryl(alkyle en $C_1$ à $C_6$) tel que phényl(alkyle en $C_1$ à $C_6$) ;

plus particulièrement, les polyacides étant choisis parmi les triacides dérivés des groupes définis en 1), en particulier en $C_5$ à $C_{20}$ ;

◦ les acides sulfocarboxyliques aromatiques ou non aromatiques comprenant au moins une fonction carboxyle -C(O)-OH et au moins une fonction sulfonique $-S(O)_2-OH$, tels que $[HO-C(O)]_y-R_d-[S(O)_2-OH]_z$ où $R_d$ est tel que défini précédemment pour les polyacides ; y et z sont des entiers supérieurs ou égaux à 1, la somme y + z est de préférence supérieure ou égale à 2, telle que égale à 3 ; les acides sulfocarboxyliques étant de préférence en $C_2$ à $C_{10}$, et le groupe acide sulfonique étant séparé du ou des groupes acides carboxyliques par une chaîne alkyle en $C_1$ à $C_6$ ou arylalkyle $C_1$ à $C_6$ polyvalente, dont la partie alkyle est linéaire ou ramifiée, éventuellement substituée par un groupe hydroxyle ;

◦ les acides phosphocarboxyliques aromatiques ou non aromatiques comprenant au moins une fonction carboxyle -C(O)-OH et au moins une fonction phosphonique $-P(O)(OH)_2$, tels que $[HO-C(O)]_y-R_d-[P(O)(OH)_2]_z$ où $R_d$ est tel que défini précédemment pour les polyacides ; y et z sont des entiers supérieurs ou égaux à 1, la somme y + z est de préférence supérieure ou égale à 2, telle que égale à 3 ; les acides phosphocarboxyliques étant en particulier en $C_2$ à $C_{10}$, et le groupe acide phosphonique étant séparé du ou des groupes acides carboxyliques par une chaîne alkyle en $C_1$ à $C_6$ ou arylalkyle $C_1$ à $C_6$ polyvalente, dont la partie alkyle est linéaire ou ramifiée et éventuellement substituée par un groupe hydroxyle.

**8.** Composition selon l'une quelconque des revendications précédentes, dans laquelle le ou les acides ii) sont choisis parmi les acides organiques.

**9.** Composition selon l'une quelconque des revendications précédentes, dans laquelle le ou les acides ii) sont choisis parmi les acides carboxyliques aromatiques ou non aromatiques comprenant au moins une fonction carboxyle -C(O)-OH.

**10.** Composition selon l'une quelconque des revendications précédentes, dans laquelle le ou les acides ii) sont choisis parmi les acides monocarboxyliques tels que définis dans la revendication 7.

**11.** Composition selon l'une quelconque des revendications précédentes, dans laquelle le ou les acides ii) sont choisis parmi les acides inorganiques.

**12.** Composition selon l'une quelconque des revendications précédentes, dans laquelle le ou les acides ii) sont choisis parmi l'acide phosphorique, l'acide glycolique, l'acide lactique, l'acide benzoïque et l'acide salicylique ; l'acide oxalique, l'acide malonique, l'acide hydroxymalonique, l'acide succinique, l'acide malique, l'acide tartrique, l'acide maléique, l'acide fumarique, l'acide itaconique, l'acide glutarique, l'acide adipique, l'acide azélaïque et l'acide sébacique, l'acide phtalique, l'acide isophtalique et l'acide téréphtalique ; l'acide sulfosuccinique, l'acide para-sulfobenzoïque, l'acide 4-sulfosalicylique, l'acide phosphoglycolique et l'acide citrique ; de préférence, le ou les acides ii) sont choisis parmi l'acide glycolique, l'acide lactique et l'acide benzoïque.

**13.** Composition selon l'une quelconque des revendications précédentes, qui comprend une teneur minimale en acides ii) tels que définis dans l'une quelconque des revendications 1 et 7 à 12 supérieure ou égale à 1 % en poids, de préférence supérieure ou égale à 2 % en poids, la teneur en acide (s) ii) qui est différente des dérivés dicarbonyliques de formule (I) tels que définis dans l'une quelconque des revendications 1 à 5 étant dans la plage encore plus préférentiellement de 2 % à 10 % en poids par rapport au poids total de la composition.

**14.** Composition selon l'une quelconque des revendications précédentes, qui a un pH supérieur ou égal à 1,7 et inférieur à 7, en particulier compris dans la plage allant de 1 à 4, plus particulièrement compris dans la plage allant de 1 à 3, et préférentiellement compris dans la plage allant de 1,7 à 3.

**15.** Composition selon l'une quelconque des revendications précédentes, **caractérisée en ce qu'**elle est aqueuse et comprend de l'eau à une concentration comprise en particulier dans la plage allant de 5 % à 98 %, plus particulièrement dans la plage allant de 5 % à 90 %, et préférentiellement dans la plage allant de 10 % à 90 % en poids par rapport au poids total de la composition.

**16.** Procédé de lissage de fibres de kératine, telles que les cheveux, en utilisant une composition comprenant

i) un ou plusieurs composés dicarbonyliques correspondant à la formule (I) ci-dessous, et/ou des hydrates de ceux-ci et/ou des sels de ceux-ci :

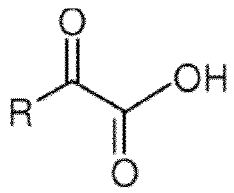

**(I)**

dans ladite formule (I) :

R représente un atome ou un groupe choisi parmi i) un hydrogène, ii) un carboxyle -C(O)-OH, iii) un alkyle en $C_1$ à $C_6$ linéaire ou ramifié qui est éventuellement substitué, de préférence par au moins un radical hydroxyle -OH, un radical carboxyle ou un radical halogène tel que Br ; iv) un phényle éventuellement substitué, v) un benzyle éventuellement substitué, iv) et v) étant de préférence éventuellement substitués par au moins un radical -OH ou -C(O)-OH ; vi) un radical indolyle et vii) un radical imidazolylméthyle et ses tautomères tels que

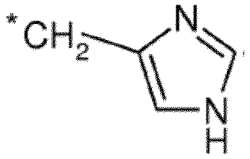

où * représente la partie liée au reste de la molécule ;

ii) un ou plusieurs acides tels que décrits dans l'une quelconque des revendications 1 et 7 à 13 ;

iii) un ou plusieurs agents alcalinisants ;

la quantité de composés dicarbonyliques de formule (I) et/ou d'hydrates de ceux-ci et/ou de sels de ceux-ci dans une composition les contenant est comprise dans la plage allant de 3 à 15 % en poids du poids total de la composition, avec iv) une étape de lissage au moyen d'un lisseur à une température d'au moins 150 °C, de préférence comprise dans la plage allant de 150 à 250 °C.

**17.** Procédé de lissage de fibres de kératine selon la revendication précédente, qui comprend l'application sur lesdites fibres de la composition comprenant :

i) un ou plusieurs composés dicarbonyliques correspondant à la formule (I) ci-dessous, et/ou des hydrates de ceux-ci et/ou des sels de ceux-ci :

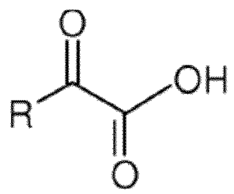

**(I)**

dans ladite formule (I) :

R représente un atome ou un groupe choisi parmi i) un hydrogène, ii) un carboxyle -C(O)-OH, iii) un alkyle en $C_1$ à $C_6$ linéaire ou ramifié qui est éventuellement substitué, de préférence par au moins un radical hydroxyle -OH, un radical carboxyle ou un radical halogène tel que Br ; iv) un phényle éventuellement substitué, v) un benzyle éventuellement substitué, iv) et v) étant de préférence éventuellement substitués par au moins un radical -OH ou -C(O)-OH ; vi) un radical indolyle et vii) un radical imidazolylméthyle et ses tautomères tels que

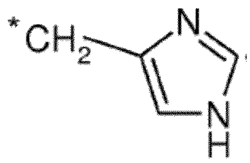

où * représente la partie liée au reste de la molécule ; les composés dicarbonyliques correspondant à la formule (I) et/ou les hydrates de ceux-ci et/ou les sels de ceux-ci étant présents dans la composition en une quantité comprise dans la plage allant de 5 à 15 % en poids du poids total de la composition ;
ii)un ou plusieurs acides différents du ou des composés i) tels que définis précédemment ; et
iii) un ou plusieurs agents alcalinisants choisis parmi i) l'ammoniaque aqueuse, ii) les carbonates ou les hydrogénocarbonates de métaux alcalins ou de métaux alcalino-terreux, tels que les carbonates ou les hydrogénocarbonates de sodium ou les carbonates ou les hydrogénocarbonates de potassium, iii) les phosphates ou les (di)hydrogénophosphates de métaux alcalins ou de métaux alcalino-terreux, iv) les hydroxydes de métaux alcalins ou de métaux alcalino-terreux, tels que les hydroxydes de sodium ou de potassium, ou des mélanges de ceux-ci, v) les alcanolamines, telles que la monoéthanolamine ou la trihydroxyéthylamine, vi) les éthylènediamines oxyéthylées et/ou oxypropylées, vii) les acides aminés et viii) les composés de formule (II) ci-dessous :

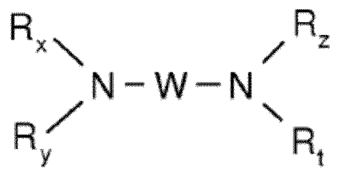
(II)

dans ladite formule (II) W représente un radical alkylène en $C_1$ à $C_6$ divalent éventuellement substitué par un ou plusieurs groupes hydroxyle ou un radical alkyle en $C_1$ à $C_6$, et/ou éventuellement interrompu par un ou plusieurs hétéroatomes tels que O, ou $NR_u$ ; $R_x$, $R_y$, $R_z$, $R_t$ et $R_u$, qui peuvent être identiques ou différents, représentent un atome d'hydrogène ou un radical alkyle en $C_1$ à $C_6$, hydroxyalkyle en $C_1$ à $C_6$, ou aminoalkyle en $C_1$ à $C_6$,

- suivie par un temps de contact compris entre 10 et 60 minutes,
- suivie par une étape de lissage au moyen d'un lisseur à une température d'au moins 150 °C, de préférence comprise dans la plage allant de 150 à 250 °C.

**18.** Utilisation d'une composition comprenant

i) un ou plusieurs composés dicarbonyliques correspondant à la formule (I) ci-dessous, et/ou des hydrates de ceux-ci et/ou des sels de ceux-ci :

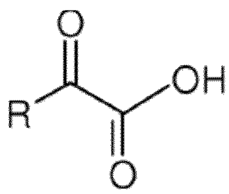
(I)

dans ladite formule (I) :
R représente un atome ou un groupe choisi parmi i) un hydrogène, ii) un carboxyle -C(O)-OH, iii) un alkyle en $C_1$ à $C_6$ linéaire ou ramifié qui est éventuellement substitué, de préférence par au moins un radical hydroxyle -OH, un radical carboxyle ou un radical halogène tel que Br ; iv) un phényle éventuellement substitué, v) un benzyle éventuellement substitué, iv) et v) étant de préférence éventuellement substitués par au moins un

radical -OH ou -C(O)-OH ; vi) un radical indolyle et vii) un radical imidazolylméthyle et sed tautomères tels que

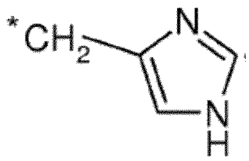

où * représente la partie liée au reste de la molécule ; les composés dicarbonyliques correspondant à la formule (I) et/ou les hydrates de ceux-ci et/ou les sels de ceux-ci étant présents dans la composition en une quantité comprise dans la plage allant de 3 à 15 % en poids du poids total de la composition ;
ii) un ou plusieurs acides différents du ou des composés i) tels que définis précédemment ; et
iii)un ou plusieurs agents alcalinisants, pour le lissage/la détente des fibres de kératine, en particulier des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011104282 A
- WO 2012105985 A
- DE 19859722
- DE 19860239


### Non-patent literature cited in the description


- CTFA dictionary. 1993